(19) Europäisches Patentamt
European Patent Office
Office européen des brevets

(11) Veröffentlichungsnummer: **0 389 901 B1**

(12) # EUROPÄISCHE PATENTSCHRIFT

(45) Veröffentlichungstag der Patentschrift: **02.03.94**

(51) Int. Cl.[5]: **C07D 285/08**, A01N 43/82

(21) Anmeldenummer: **90105075.7**

(22) Anmeldetag: **17.03.90**

(54) **Thiadiazol-substituierte Acrylsäureester und neue Zwischenprodukte.**

(30) Priorität: **31.03.89 DE 3910358**

(43) Veröffentlichungstag der Anmeldung:
**03.10.90 Patentblatt 90/40**

(45) Bekanntmachung des Hinweises auf die Patenterteilung:
**02.03.94 Patentblatt 94/09**

(84) Benannte Vertragsstaaten:
**BE CH DE DK FR GB IT LI NL**

(56) Entgegenhaltungen:
**DE-A- 2 037 474**
**DE-A- 2 635 568**
**DE-A- 3 228 131**

(73) Patentinhaber: **BAYER AG**

**D-51368 Leverkusen(DE)**

(72) Erfinder: **Heinemann, Ulrich, Dr.**
**Feldstrasse 18**
**D-5653 Leichlingen 1(DE)**
Erfinder: **Kleefeld, Gerd, Dr.**
**Otto-Hahn-Strasse 87**
**D-4000 Düsseldorf 13(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

**EP 0 389 901 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Thiadiazol-substituierte Acrylsäureester, mehrere Verfahren zu ihrer Herstellung, ihre Verwendung zur Bekämpfung von Schädlingen und neue Zwischenprodukte.

Es ist bekannt, daß bestimmte substituierte Acrylsäureester, wie beispielsweise die Verbindung 3-Methoxy-2-(2-methylphenyl)-acrylsäuremethylester fungizide Eigenschaften besitzen (vgl. z.B. EP 178 826).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue Thiadiazol-substituierte Acrylsäureester der allgemeinen Formel (I),

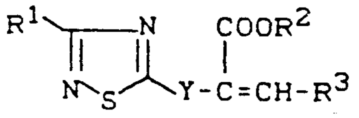

(I)

in welcher

$R^1$ für Wasserstoff, für unsubstituiertes oder ein- bis mehrfach, gleich oder verschieden substituiertes, geradkettiges oder verzweigtes Alkyl mit 1 bis 8 Kohlenstoffatomen steht, wobei folgende Substituenten in Frage kommen:
Fluor, Chlor, Brom, Jod, jeweils unsubstituiertes oder ein- bis fünffach, gleich oder verschieden substituiertes Arylthio und Aryloxy mit jeweils 6 bis 10 Kohlenstoffatomen im Arylteil, wobei als Arylsubstituenten die weiter unten in der Definition von $R^1$ aufgeführten Arylsubstituenten genannt seien;

$R^1$ weiterhin für geradkettiges oder verzweigtes Alkenyl mit 2 bis 8 Kohlenstoffatomen, für jeweils unsubstituiertes oder im Arylteil einfach bis mehrfach, gleich oder verschieden substituiertes Aralkyl mit 1 bis 6 Kohlenstoffatomen im geradkettigen oder verzweigten Alkylteil, Aralkenyl mit 2 bis 6 Kohlenstoffatomen im geradkettigen oder verzweigten Alkenylteil oder für Aryl mit 6 bis 10 Kohlenstoffatomen im jeweiligen Arylteil steht, wobei als Arylsubstituenten jeweils infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, geradkettiges oder verzweigtes Alkoxycarbonyl mit 1 bis 4 Kohlenstoffatomen oder Alkoximinoalkyl mit jeweils 1 bis 6 Kohlenstoffatomen in den einzelnen Alkylteilen, Cycloalkyl mit 3 bis 8 Kohlenstoffatomen, zweifach verknüpftes Alkandiyl mit 3 bis 5 Kohlenstoffatomen, jeweils unsubstituiertes oder im Arylteil einfach bis mehrfach, gleich oder verschieden durch Halogen, Alkyl, Alkoxy, Alkylthio, Halogenalkyl, Halogenalyoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 8 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil;

$R^1$ außerdem für einen gegebenenfalls einfach bis mehrfach, gleich oder verschieden substituierten 5- oder 6-gliedrigen Heteroarylrest mit 1 bis 3 gleichen oder verschiedenen Heteroatomen - insbesondere Stickstoff, Sauerstoff und/oder Schwefel - steht, wobei als Substituenten die oben genannten Arylsubstituenten infrage kommen,

$R^2$ für geradkettiges oder verzweigtes Alkyl mit 1 bis 6 Kohlenstoffatomen steht,

$R^3$ für Dialkylamino mit jeweils 1 bis 6 Kohlenstoffatomen in den einzelnen geradkettigen oder verzweigten, gleichen oder verschiedenen Alkylteilen steht oder für einen Rest -Z-$R^4$ steht,

Y für Sauerstoff, Schwefel oder für einen Rest

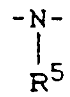

steht,

$R^4$ für geradkettiges oder verzweigtes Alkyl mit 1 bis 6 Kohlenstoffatomen oder für unsubstituiertes oder im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^5$ für Wasserstoff oder für geradkettiges oder verzweigtes Alkyl mit 1 bis 6 Kohlenstoffatomen steht und

Z für Sauerstoff oder Schwefel steht,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Thiadiazol-substituierten Acrylsäureester der allgemeinen Formel (I),

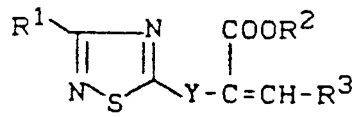

(I)

in welcher

$R^1$, $R^2$ und $R^3$ die oben angegebene Bedeutung haben

nach einem der im Folgenden beschriebenen Verfahren erhält:

(a) Man erhält Thiadiazol-substituierte Acrylsäureester der Formel (Ia),

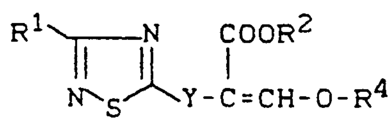

(Ia)

in welcher

$R^1$, $R^2$, $R^4$ und Y die oben angegebene Bedeutung haben,

wenn man Hydroxyacrylsäureester oder deren Alkalimetallsalze der Formel (II),

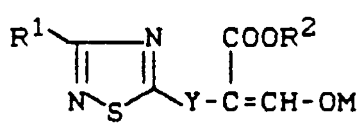

(II)

in welcher

M für Wasserstoff oder für ein Alkalimetallkation steht und

$R^1$, $R^2$ und Y die oben angegebene Bedeutung haben,

mit Alkylierungsmitteln der Formel (III),

$R^4$-$E^1$ (III)

in welcher

$E^1$ für eine elektronenanziehende Abgangsgruppe steht und

$R^4$ die oben angegebene Bedeutung hat,
gegebenenfalls in Gegenwart eines Verdünnungsmittels und gegebenenfalls in Gegenwart eines Reaktionshilfsmittels umsetzt;
oder
(b) man erhält Thiadiazol-substituierte Acrylsäureester der Formel (Ib),

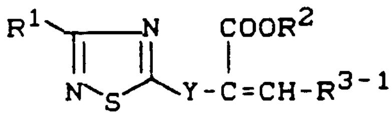
(Ib)

in welcher
$R^{3-1}$ für Dialkylamino steht und
$R^1$, $R^2$ und Y die oben angegebene Bedeutung haben,
wenn man substituierte Essigsäureester der Formel (IV),

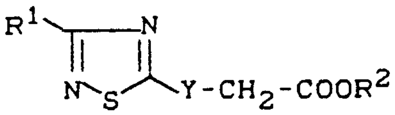
(IV)

in welcher
$R^1$, $R^2$ und Y die oben angegebene Bedeutung haben,
mit Formamidderivaten der Formel (V),

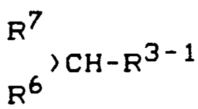
(V)

in welcher
$R^6$ und $R^7$ unabhängig voneinander jeweils für Alkoxy oder Dialkylamino stehen und
$R^{3-1}$ die oben angegebene Bedeutung hat,
gegebenenfalls in Gegenwart eines Verdünnungsmittels umsetzt;
oder
(c) man erhält Thiadiazol-substituierte Acrylsäureester der Formel (Ic),

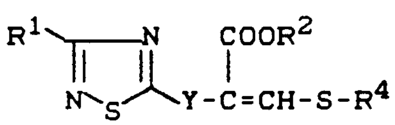
(Ic)

in welcher
$R^1$, $R^2$, $R^4$ und Y die oben angegebene Bedeutung haben,
wenn man substituierte Acrylsäureester der Formel (VI),

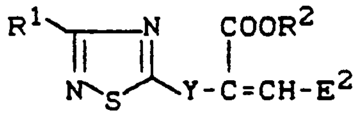

(VI)

in welcher

$E^2$ für eine elektronenanziehende Abgangsgruppe steht und

$R^1$, $R^2$ und Y die oben angegebene Bedeutung haben,

mit Thiolen der Formel (VII)

$R^4$-SH (VII)

in welcher

$R^4$ die oben angegebene Bedeutung hat,

gegebenenfalls in Gegenwart eines Verdünnungsmittels und gegebenenfalls in Gegenwart eines Reaktionshilfsmittels umsetzt.

Schließlich wurde gefunden, daß die neuen Thiadiazol-substituierten Acrylsäureester der allgemeinen Formel (I) eine gute Wirkung gegen Schädlinge besitzen.

Überraschenderweise zeigen die erfindungsgemäßen Thiadiazol-substituierten Acrylsäureester der allgemeinen Formel (I) z.B. eine erheblich bessere fungizide Wirksamkeit als die aus dem Stand der Technik bekannten Acrylsäureester, wie beispielsweise die Verbindung 3-Methoxy-2-(2-methylphenyl)-acrylsäuremethylester, welche chemisch und wirkungsmäßig naheliegende Verbindungen sind.

Bevorzugte Substituenten bzw. Bereiche der in den oben und nachstehend erwähnten Formeln aufgeführten Reste werden im folgenden erläutert:

Für unsubstituiertes oder substituiertes Alkyl in den Definitionen von $R^1$, $R^2$, $R^4$ und $R^5$ in den allgemeinen Formeln, steht geradkettiges oder verzweigtes Alkyl mit vorzugsweise 1 bis 10, besonders bevorzugt 1 bis 8 und insbesondere 1 bis 4, Kohlenstoffatomen. Beispielhaft seien unsubstituiertes oder substituiertes Methyl, Ethyl, n-Propyl, i-Propyl, n-Butyl, sec-Butyl, i-Butyl, t-Butyl, n-Pentyl, i-Pentyl und t-Pentyl genannt.

Für den Begriff unsubstituiertes oder substituiertes Alkenyl in den Definitionen von $R^1$ in den allgemeinen Formeln steht geradkettiges oder verzweigtes Alkenyl mit vorzugsweise 2 bis 8, besonders bevorzugt 2 bis 6 und insbesondere 2 bis 4, ganz besonders bevorzugt 3 Kohlenstoffatomen. Beispielhaft seien unsubstituiertes oder substituiertes Vinyl, Allyl, Propenyl-(2), Butenyl-(1), 2-Butenyl, 3-Butenyl und 1-Methallyl genannt.

Dialkylamino in der Definition von $R^3$ steht für eine Aminogruppe mit 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n- und i-Propyl genannt seien. Beispielhaft seien Dimethylamino, Diethylamino, Di-n-propylamino und Di-i-propylamino aufgeführt.

Unter dem Begriff unsubstituiertes oder substituiertes Aryl in der Definition von $R^1$ in den allgemeinen Formeln ist Aryl mit vorzugsweise 6 bis 10 Kohlenstoffatomen im Arylteil zu verstehen. Beispielhaft seien unsubstituiertes oder substituiertes Phenyl oder Naphthyl, insbesondere Phenyl genannt.

Unsubstituiertes oder substituiertes Aralkyl in den Definitionen von $R^1$ und $R^4$ enthält vorzugsweise 1 bis 6 Kohlenstoffatome im geradkettigen oder verzweigten Alkylteil und vorzugsweise Phenyl als Arylteil. Als Aralkylgruppen seien beispielhaft Benzyl und Phenethyl genannt.

Unsubstituiertes oder substituiertes Aralkenyl in der Definition von $R^1$ enthält vorzugsweise 2 bis 6, insbesondere 2 bis 4, ganz besonders bevorzugt 3 Kohlenstoffatome im geradkettigen oder verzweigten Alkenylteil und vorzugsweise Phenyl als Arylteil. Als Aralkenylgruppe sei beispielhaft Styryl genannt.

Heteroaryl in der Definition von $R^1$ steht im allgemeinen für einen 5- oder 6-gliedrigen Ring, der ein oder mehrere Heteroatome, bevorzugt 1 bis 3 gleiche oder verschiedene Heteroatome, enthält. Als Heteroatome seien vorzugsweise Sauerstoff, Schwefel und Stickstoff genannt; beispielhaft seien genannt: Pyridyl, Thienyl oder Furyl.

Speziell die Substituenten für die Arylreste als solche oder in Zusammensetzungen wie Arylalkyl, Aryloxy, Arylthio, Aralkyloxy und für die heterocyclischen Ringe wie Heteroarylalkyl und Heteroaryl haben die im folgenden angegebenen Bedeutungen.

Halogen steht im allgemeinen als Substituent der Reste für Fluor, Chlor, Brom und Iod, bevorzugt für Fluor, Chlor und Brom, besonders bevorzugt für Fluor und Chlor.

Alkyl steht im allgemeinen als Substituent der Reste selbst oder in Zusammensetzungen wie Alkoximinoalkyl für geradkettiges oder verzweigtes Alkyl, bevorzugt mit 1 bis 6, besonders bevorzugt mit 1 bis 4 Kohlenstoffatomen, ganz besonders bevorzugt sind Methyl, Ethyl und t-Butyl. Die beispielhafte Aufzählung entspricht der weiter oben gegebenen.

Alkoxy steht im allgemeinen als Substituent der Reste selbst oder in Zusammensetzungen wie Alkoximinoalkyl für geradkettiges oder verzweigtes Alkoxy mit 1 bis 6, besonders bevorzugt 1 bis 3 Kohlenstoffatomen je Alkylrest; beispielhaft seien genannt: Methoxy, Ethoxy, n- und i-Propoxy, n-, i-, s- und t-Butoxy, n-Hexoxy und i-Hexoxy.

Alkylthio steht im allgemeinen als Substituent in den Resten für geradkettiges oder verzweigtes Alkylthio mit vorzugsweise 1 bis 6 Kohlenstoffatomen, beispielsweise sind darunter die folgenden Gruppen zu verstehen: Methylthio-, Ethylthio-, Propylthio-, Butylthio-, Pentylthio sowie ihre Isomeren, wie z. B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Halogenalkyl und Halogenalkoxy stehen im allgemeinen als Substituenten in den Resten für geradkettige oder verzweigte Reste mit je 1 bis 4 Kohlenstoffatomen, besonders bevorzugt mit 1 oder 2 Kohlenstoffatomen und jeweils 1 bis 9, beziehungsweise 1 bis 5, gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Halogenalkylthio steht im allgemeinen als Substituent in den Resten für geradkettige oder verzweigte Reste mit je 1 bis 4 Kohlenstoffatomen, besonders bevorzugt mit 1 oder 2 Kohlenstoffatomen und jeweils 1 bis 9, beziehungsweise 1 bis 5, gleichen oder verschiedenen Halogenatomen wie unter Halogen definiert; beispielhaft seien genannt: Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

Alkoxycarbonyl steht im allgemeinen als Substituent in den Resten für geradkettiges oder verzweigtes Alkoxycarbonyl mit 1 bis 4, vorzugsweise 1 oder 2 Kohlenstoffatomen im Alkoxyrest; beispielhaft seien genannt: Methoxycarbonyl, Ethoxycarbonyl, n-Propoxycarbonyl, i-Propoxycarbonyl, n-, i-, s- und t-Butoxycarbonyl.

Cycloalkyl steht im allgemeinen als Substituent in den Resten für Cycloalkyl mit vorzugsweise 3 bis 8, insbesondere 3, 5 oder 6 Kohlenstoffatomen. Beispielhaft seien unsubstituiertes oder substituiertes Cyclopropyl, Cyclobutyl, Cyclopentyl, Cyclohexyl und Cycloheptyl genannt.

Die erfindungsgemäßen Thiadiazol-substituierten Acrylsäureester sind durch die Formel (I) allgemein definiert. Bevorzugt sind Verbindungen der Formel (I), bei welchen

$R^1$ für Wasserstoff, Methyl, Ethyl, n- oder i-Propyl, n-, i-, s- oder t-Butyl, ein- bis sechsfach, gleich oder verschieden durch Fluor und Chlor substituiertes geradkettiges oder verzweigtes Alkyl mit 1 bis 4 Kohlenstoffatomen oder für geradkettiges oder verzweigtes Alkyl mit 1 bis 4 Kohlenstoffatomen steht, welches durch jeweils unsubstituiertes oder ein- bis dreifach, gleich oder verschieden substituiertes Phenylthio oder Phenyloxy substituiert ist, wobei als Phenylsubstituenten die weiter unten in der Definition von $R^1$ aufgeführten Substituenten genannt seien;

$R^1$ für Allyl, n- oder i-Butenyl, für jeweils gegebenenfalls im Arylteil oder im Heteroarylteil ein- bis dreifach, gleich oder verschieden substituiertes Benzyl, Phenylethyl, Phenylethenyl, Phenyl, Naphthyl, Pyridyl, Thienyl oder Furyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, But-2-en-di-1,4-yl, 1,4-Butandiyl oder jeweils unsubstituiertes oder im Phenylteil einbis dreifach, gleich oder verschieden durch Fluor, Chlor, Brom, Methyl, Ethyl, Methoxy, Ethoxy, Methylthio, Trifluormethyl, Difluormethoxy, Trifluormethoxy und Trifluormethylthio substituiertes Phenyl, Benzyl, Phenoxy oder Benzyloxy,

$R^2$ für Methyl, Ethyl, n- oder i-Propyl, n-, i-, s- oder t-Butyl steht,

$R^3$ für Dialkylamino mit jeweils 1 bis 4 Kohlenstoffatomen in den einzelnen geradkettigen oder verzweigten, gleichen oder verschiedenen Alkylteilen steht oder für einen Rest -Z-$R^4$ steht,

Y für Sauerstoff, Schwefel oder für einen Rest

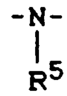

steht,

$R^4$ für Methyl, Ethyl, n- oder i-Propyl, n-, i-, s- oder t-Butyl oder für unsubstituiertes oder ein- bis dreifach, gleich oder verschieden substituiertes Benzyl steht, wobei als Substituenten die bei $R^1$ genannten infrage kommen;

$R^5$ für Wasserstoff, Methyl, Ethyl, n- oder i-Propyl, n-, i-, s- oder t-Butyl und

Z für Sauerstoff oder Schwefel stehen.

Ganz besonders bevorzugt sind Verbindungen der Formel (I), bei welchen

$R^1$ für Wasserstoff, Methyl, Ethyl, n- oder i-Propyl, t-Butyl, ein- bis fünffach, gleich oder verschieden durch Fluor und Chlor substituiertes Alkyl mit 1 oder 2 Kohlenstoffatomen oder für Alkyl mit 1 oder 2 Kohlenstoffatomen steht, welches durch jeweils unsubstituiertes oder ein- oder zweifach, gleich oder verschieden substituiertes Phenylthio und Phenyloxy substituiert ist, wobei als Phenylsubstituenten die weiter unten in der Definition von $R^1$ aufgeführten Substituenten genannt seien oder

$R^1$ für jeweils gegebenenfalls ein- bis dreifach, gleich oder verschieden substituiertes Phenyl oder Naphthyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und Ethyl substituiertes Phenyl, Phenoxy, Benzyl oder Benzyloxy,

$R^2$ für Methyl oder Ethyl steht,

$R^3$ für Dimethylamino, Diethylamino oder für einen Rest -Z-$R^4$ steht,

Y für Sauerstoff, Schwefel oder für einen Rest

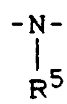

steht,

$R^4$ für Methyl, Ethyl, n- oder i-Propyl oder Benzyl steht,

$R^5$ für Wasserstoff, Methyl oder Ethyl steht und

Z für Sauerstoff oder Schwefel steht.

Insbesonders bevorzugt sind Verbindungen der Formel (I), bei welchen

$R^1$ für Wasserstoff, Methyl, Ethyl, n- oder i-Propyl, t-Butyl, ein- bis dreifach, gleich oder verschieden durch Fluor und Chlor substituiertes Methyl oder für jeweils gegebenenfalls einfach oder zweifach, gleich oder verschieden substituiertes Phenyl, Phenyloxymethyl oder Phenylthiomethyl steht, wobei als Substituenten infrage kommen:
Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Cyclopentyl, 1,3-Propandiyl, Methoximinoethyl oder gegebenenfalls ein- oder zweifach, gleich oder verschieden durch Fluor, Chlor, Brom oder Methyl substituiertes Phenyl, Phenoxy, Benzyl oder Benzyloxy,

$R^2$ für Methyl oder Ethyl steht,

$R^3$ für Methoxy, Ethoxy, Methylthio oder Dimethylamino steht und

Y für einen N-Methyl-Rest oder Schwefel steht.

Im einzelnen seien außer den bei den Herstellungsbeispielen genannten Verbindungen die in der nachfolgenden Tabelle 1 aufgeführten Thiadiazol-substituierten Acrylsäureester der allgemeinen Formel (I) genannt:

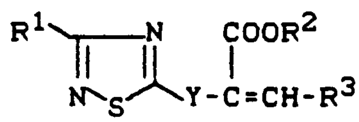 (I)

Tabelle 1

| $R^1$ | $R^2$ | $R^3$ | Y |
|---|---|---|---|
| 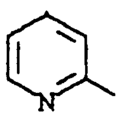 | $CH_3$ | $OCH_3$ | $N-CH_3$ |
| 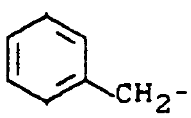 | $CH_3$ | $OCH_3$ | $N-CH_3$ |
| 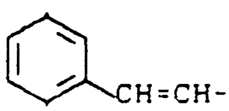 | $CH_3$ | $OCH_3$ | $N-CH_3$ |
| 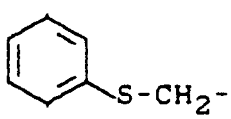 | $CH_3$ | $OCH_3$ | $N-CH_3$ |

Verwendet man beispielsweise N-(3-Phenyl-1,2,4-thiadiazol-5-yl)-N-methyl-2-amino-3-hydroxy-acrylsäuremethylester und Dimethylsulfat als Ausgangsstoffe, so läßt sich der Reaktionsablauf des erfindungsgemäßen Verfahrens (a) durch das folgende Formelschema darstellen:

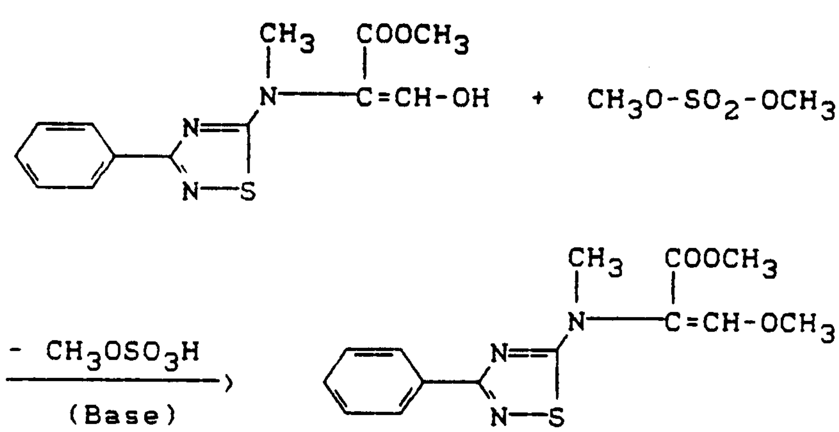


Verwendet man beispielsweise N-(3-Trifluormethyl-1,2,4-thiadiazol-5-yl)-N-methyl-glycin-methylester und Dimethylformamiddimethylacetal als Ausgangsstoffe, so läßt sich der Reaktionsablauf des erfindungsgemäßen

Verfahrens (b) durch das folgende Formelschema darstellen:

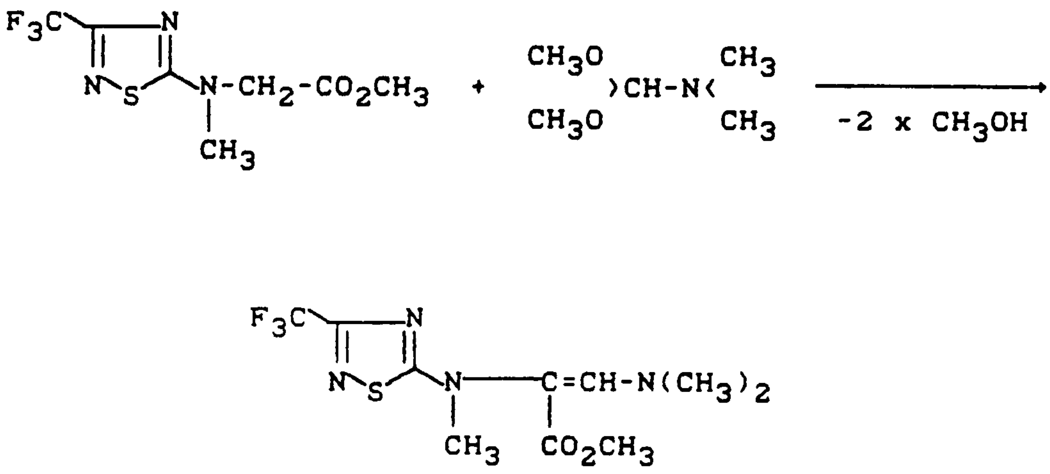


Verwendet man beispielsweise N-(3-Phenyl-1,2,4-thiadiazol-5-yl)-N-methyl-2-amino-3-methylsulfonyloxy-acrylsäuremethylester und Methylmercaptan als Ausgangstoffe, so läßt sich der Reaktionsablauf des erfindungsgemäßen Verfahrens (c) durch das folgende Formelschema darstellen:

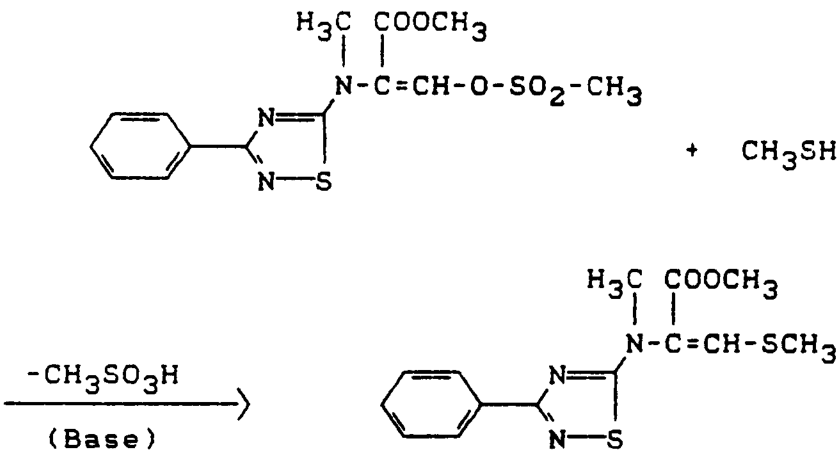


Die zur Durchführung des erfindungsgemäßen Verfahrens (a) als Ausgangsstoffe benötigten Hydroxyacrylsäureester oder deren Alkalimetallsalze sind durch die Formel (II) allgemein definiert. In dieser Formel (II) stehen $R^1$, $R^2$ und Y vorzugsweise für diejenigen Reste, die bereits im Zusammenhang mit der Beschreibung der erfindungsgemäßen Stoffe der Formel (I) als bevorzugt für diese Substituenten genannt wurden.

M steht vorzugsweise für Wasserstoff oder für ein Natrium-, Kalium- oder Lithiumkation.

Die Hydroxyacrylsäureester der Formel (II) sind noch nicht bekannt und ebenfalls Gegenstand der Erfindung.

Man erhält sie, wenn man substituierte Essigsäureester der Formel (IV),

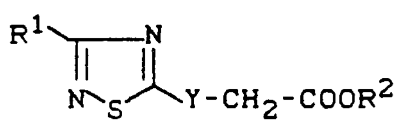
(IV)

in welcher

$R^1$, $R^2$ und Y die oben angegebene Bedeutung haben,

mit Ameisensäureestern der Formel (VIII),

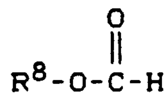 (VIII)

in welcher

$R^8$ für Alkyl, insbesondere für Methyl oder Ethyl steht,

gegebenenfalls in Gegenwart eines Verdünnungsmittels, wie beispielsweise Dimethylformamid, und gegebenenfalls in Gegenwart eines basischen Reaktionshilfsmittels, wie beispielsweise Natriumhydrid, bei Temperaturen zwischen - 20 °C und + 50 °C umsetzt und gegebenenfalls anschließend mit einer Säure, wie beispielsweise Salzsäure, hydrolysiert.

Ameisensäureester der Formel (VIII) sind allgemein bekannte Verbindungen der organischen Chemie.

Die zur Durchführung des erfindungsgemäßen Verfahrens (a) weiterhin als Ausgangsstoffe benotigten Alkylierungsmittel sind durch die Formel (III) allgemein definiert. In dieser Formel (III) steht $R^4$ vorzugsweise für diejenigen Reste, die bereits im Zusammenhang mit der Beschreibung der erfindungsgemäßen Stoffe der Formel (I) als bevorzugt für diesen Substituenten genannt wurden.

$E^1$ steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten substituierten Essigsäureester sind durch die Formel (IV) allgemein definiert. In dieser Formel (IV) stehen $R^1$, $R^2$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Essigsäureester der Formel (IV) sind teilweise bekannt (vgl. z.B. US 4 207 090, GB 1 574 430, DE 2 050 346 und Yakugaku Zasshi, 88, 1437-49 [1968]).

Noch nicht bekannt und ebenfalls Gegenstand der Erfindung sind substituierte Essigsäureester der Formel (IVa),

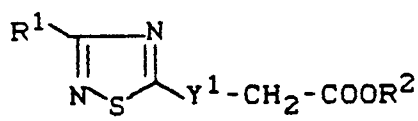 (IVa)

in welcher

$Y^1$ für Sauerstoff oder für einen N-Alkylrest, vorzugsweise mit 1 bis 4 Kohlenstoffatomen, insbesondere für einen -N-Methylrest oder für einen -N-Ethylrest steht und

$R^1$ und $R^2$ die oben angegebene Bedeutung haben, ausgenommen die Verbindungen N-Methyl-N-[3-(trichlormethyl)-1,2,4-thiadiazol-5-y]-glycinethylester und N-Methyl-N-[3-(trifluormethyl)-1,2,4-thiadiazol-5-yl]-glycinethylester (vgl. US 4 207 090).

Man erhält die neuen substituierten Essigsäureester der Formel (IVa), indem man 1,2,4-Thiadiazolderivate der Formel (IX),

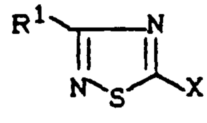 (IX)

in welcher

$R^1$ die oben angegebene Bedeutung hat und

X für Halogen, vorzugsweise Fluor oder Chlor, insbesondere Chlor, steht

mit Essigsäureesterderivaten der Formel (X)

$HY^1-CH_2-COOR^2$ (X)

in welcher

$R^2$ und $Y^1$ die oben angegebene Bedeutung haben oder deren Hydrochloride,

gegebenenfalls in Gegenwart eines Verdünnungsmittels und gegebenenfalls in Gegenwart einer Base umsetzt.

Das erfindungsgemäße Verfahren zur Herstellung der neuen substituierten Essigsäureester der Formel (IVa) wird vorzugsweise unter Verwendung von Verdünnungsmitteln durchgeführt. Als solche kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren zur Herstellung der neuen Ausgangsverbindungen wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen - 50 °C und + 200 °C, vorzugsweise bei Temperaturen zwischen - 20 °C und + 150 °C.

Zur Durchführung des erfindungsgemäßen Verfahrens setzt man die Ausgangskomponenten und die Basen im allgemeinen in äquivalentem Verhältnis ein. Ein Überschuß der einen oder anderen Komponente bringt meist keine wesentlichen Vorteile. Die Reaktionspartner werden im allgemeinen in einem der oben angeführten Lösungsmittel in Gegenwart der Base vereinigt und bei der entsprechenden Temperatur eine oder mehrere Stunden gerührt. Die Aufarbeitung des Reaktionsgemisches erfolgt nach üblichen Methoden.

Die Thiadiazol-Derivate der Formel (IX) sind bekannt oder erhältlich in Analogie zu bekannten Verfahren (vgl. z.B. DE 2 242 185, DE 3 228 147, Chem. Ber. 90, 182 [1957] ibid. 90, 892 [1957]).

Die Essigsäureester-Derivate der Formel (X) sind allgemein bekannte Verbindungen der organischen Chemie.

Die zur Durchführung des erfindungsgemäßen Verfahrens (b) weiterhin als Ausgangsstoffe benötigten Formamidderivate sind durch die Formel (V) allgemein definiert. In dieser Formel (V) steht $R^{3-1}$ vorzugsweise für Dialkylamino mit jeweils 1 bis 6, insbesondere mit 1 bis 4 Kohlenstoffatomen in den einzelnen geradkettigen oder verzweigten Alkylteilen. $R^{3-1}$ steht ganz besonders bevorzugt für Dimethylamino oder Diethylamino,

$R^6$ und $R^7$ stehen vorzugsweise unabhängig voneinander jeweils für geradkettiges oder verzweigtes Alkoxy mit 1 bis 4 Kohlenstoffatomen insbesondere für Methoxy oder Ethoxy oder für einen Dialkylaminorest mit jeweils 1 bis 6, insbesondere mit 1 bis 4 Kohlenstoffatomen in den einzelnen geradkettigen oder verzweigten Alkylteilen.

Die Formamid-Derivate der Formel (V ) sind allgemein bekannte Verbindungen der organischen Chemie.

Die zur Durchführung des erfindungsgemäßen Verfahrens (c) als Ausgangsstoffe benötigten substituierten Acrylsäureester sind durch die Formel (VI) allgemein definiert. In dieser Formel (VI) stehen $R^1$, $R^2$ und Y vorzugsweise für diejenigen Reste, die bereits im Zusammenhang mit der Beschreibung der erfindungsgemäßen Stoffe der Formel (I) als bevorzugt für diese Substituenten genannt wurden.

$E^2$ steht vorzugsweise für einen geeigneten Acyloxy- oder Sulfonyloxyrest, insbesondere für einen Acetoxy-, einen Methansulfonyloxy- oder einen p-Toluolsulfonyloxyrest.

Die substituierten Acrylsäureester der Formel (VI) sind noch nicht bekannt.

Man erhält sie, wenn man Hydroxyacrylsäureester der Formel (II),

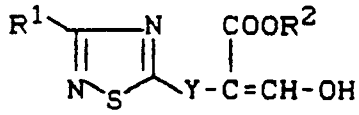

(II)

in welcher

$R^1$, $R^2$ und Y die oben angegebene Bedeutung haben,

mit Säurechloriden der Formel (XI),

$R^9$-Cl (XI)

in welcher

$R^9$ für einen Acyl- oder Sulfonylrest, insbesondere für einen Acetyl-, einen Methansulfonyl- oder einen p-Toluolsulfonylrest steht,

gegebenenfalls in Gegenwart eines Verdünnungsmittels, wie beispielsweise Dichlormethan, und gegebenenfalls in Gegenwart eines Säurebindemittels, wie beispielsweise Triethylamin oder Pyridin, bei Temperaturen zwischen - 20 °C und + 120 °C umsetzt.

Säurechloride der Formel (XI) sind allgemein bekannte Verbindungen der organischen Chemie.

Die zur Durchführung des erfindungsgemäßen Verfahrens (c) weiterhin als Ausgangsstoffe benötigten Thiole sind durch die Formel (VII) allgemein definiert. In dieser Formel (VII) steht $R^4$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Thiole der Formel (VII) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Nitrile, wie Acetonitril oder Propioni-tril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethyl-ammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (a) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen - 30 °C und + 120 °C, vorzugsweise bei Temperaturen zwischen - 20 °C und + 60 °C.

Zur Durchführung des erfindungsgemäßen Verfahrens (a) setzt man pro Mol an 3-Hydroxyacrylsäureester oder eines entsprechenden Alkalimetallsalzes der Formel (II) im allgemeinen 1.0 bis 10.0 Mol, vorzugsweise 1.0 bis 5.0 Mol an Alkylierungsmittel der Formel (III) und gegebenenfalls 1.0 bis 5.0 Mol, vorzugsweise 1.0 bis 2.0 Mol an Reaktionshilfsmittel ein.

Dabei ist es auch möglich, die als Ausgangsverbindungen zur Durchführung des erfindungsgemäßen Verfahrens (a) benötigten 3-Hydroxyacrylsäureester oder deren Alkalimetallsalze der Formel (II) in einer vorgelagerten Reaktion direkt im Reaktionsgefäß herzustellen und direkt aus dem Reaktionsgemisch heraus ohne Isolierung mit dem Alkylierungsmittel der Formel (III) gemäß dem erfindungsgemäßen Verfahren (a)

weiter umzusetzen ("Eintopfverfahren").
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether.

Es ist jedoch auch möglich, das erfindungsgemäße Verfahren (b) ohne Zusatz eines Verdünnungsmittels durchzuführen.

Die Reaktionstemperaturen können bei der Durchführung des erfindungsgemäßen Verfahrens (b) in einem größeren Bereich variiert werden. Im allgemeinen arbeitet man bei Temperaturen zwischen - 20 °C und + 220 °C, vorzugsweise bei Temperaturen zwischen 0 °C und 200 °C.

Zur Durchführung des erfindungsgemäßen Verfahrens (b) setzt man pro Mol an substituiertem Essigsäureester der Formel (IV) im allgemeinen 1.0 bis 30.0 Mol, vorzugsweise 1.0 bis 15.0 Mol an Formamid-Derivat der Formel (V) ein. Die Reaktionsdurchführung, Aufarbeitung und Isolierung der Reaktionsprodukte erfolgt nach allgemein üblichen Methoden (vgl. hierzu auch G. Mathieu; J. Weill-Raynal "Formation of C-C-Bonds", Vol. I; p. 229-244; Thieme Verlag Stuttgart 1973).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (c) wird vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, oder tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen - 20 °C und 180 °C, vorzugsweise bei Temperaturen zwischen 0 °C und 150 °C.

Das erfindungsgemäße Verfahren kann in Abhängigkeit vom Siedepunkt der verwendeten Reaktionspartner, beispielsweise beim Einsatz von niedrigsiedenden Thiolen der Formel (VII) gegebenenfalls auch unter Druck durchgeführt werden.
Vorzugsweise arbeitet man dann bei dem Druck, der sich beim Erhitzen auf die erforderliche Reaktionstemperatur unter den Reaktionsbedingungen einstellt.

Zur Durchführung des erfindungsgemäßen Verfahrens (c) setzt man pro Mol an substituiertem Acrylsäureester der Formel (VI) im allgemeinen 1.0 bis 20.0 Mol, vorzugsweise 1.0 bis 5.0 Mol an Thiol der Formel (VII) und gegebenenfalls 1.0 bis 5.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach allgemein üblichen Methoden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch z.B. als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;

Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise
Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur protektiven Bekämpfung von Getreidekrankheiten, wie beispielsweise gegen den Erreger der Braunspelzigkeit des Weizens (Leptosphaeria nodorum) oder zur protektiven Bekämpfung von Reiskrankheiten, wie beispielsweise gegen den Erreger der Reisfleckenkrankheit (Pyricularia oryzae) einsetzen.

Darüberhinaus zeigen einige der erfindungsgemäßen Wirkstoffe außerdem eine fungizide Wirkung gegen echte Mehltaupilze u.a. an Getreide, sowie an Apfelschorf.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden.Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die Herstellung und die Verwendung der erfindungsgemäßen Wirkstoffe geht aus den nachfolgenden Beispielen hervor.

Herstellungsbeispiele

Beispiel 1:

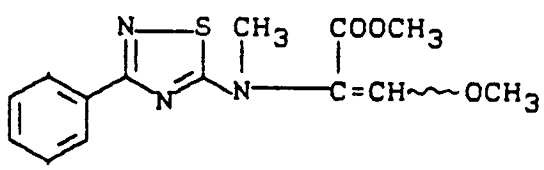

Verfahren (a)-Eintopfvariante

Zu einer Suspension von 3.3 g (0.11 Mol) einer 80%igen Natriumhydridsuspension in Mineralöl und 150 ml abs. Dimethylformamid tropft man bei 5-10 °C innerhalb von 90 Min. ein Gemisch von 130.0 g (2.17 Mol) Ameisensäuremethylester und 11.5 g (0.044 Mol) N-(3-Phenyl-1,2,4-thiadiazol-5-yl)-N-methyl-2-aminoessigsäuremethylester. Nach Abklingen der anfangs heftigen Reaktion läßt man zuerst auf Raumtemperatur kommen und rührt dann 2h bei 30 °C nach.

Anschließend gibt man zu dem nicht isolierten N-(3-Phenyl-1,2,4-thiadiazol-5-yl)-N-methyl-2-amino-3-hydroxy-acrylsäuremethylester 13.9 g (0.11 Mol) Dimethylsulfat und rührt über Nacht bei Raumtemperatur. Zur Aufarbeitung gießt man auf 2 l Eiswasser, extrahiert 3x mit Diethylether und trocknet die organische Phase mit Natriumsulfat. Nach Abdestillieren des Lösungsmittels wird das Rohprodukt an Kieselgel chromatographiert (Laufmittel, Toluol/Propanol 10:3).

Man erhält 6.0 g (44% der Theorie) N-(3-Phenyl-1,2,4-thiadiazol-5-yl)-N-methyl-2-amino-3-methoxy-acrylsäuremethylester.

Beispiel 2:

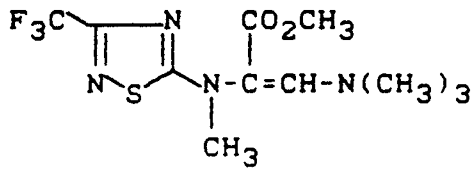

Verfahren (b)

12.8 g (0.05 Mol) N-(3-Trifluormethyl-1,2,4-thiadiazol-5-yl)-N-methyl-glycinmethylester und 14.7 g (0.10 Mol) Dimethylformamiddiethylacetal werden 48 Stunden unter Rückfluß erhitzt. Nach dem Abkühlen wird unter vermindertem Druck eingeengt, der Rückstand mit Ether verrührt und die ausgefallenen Kristalle abgesaugt.

Man erhält 10.0 g (65% der Theorie) N-(3-Trifluormethyl-1,2,4-thiadiazol-5-yl)-N-methyl-2-amino-3-N′,N′-dimethylamino-acrylsäuremethylester vom Schmelzpunkt 130-131 °C.

In analoger Weise zu den in den Beispielen 1 und 2 beschriebenen Methoden und unter Berücksichtigung der Angaben in den Beschreibungen zu den erfindungsgemäßen Verfahren, werden die in der nachfolgenden Tabelle 2 aufgeführten Endprodukte der Formel (I) erhalten.

Tabelle 2:

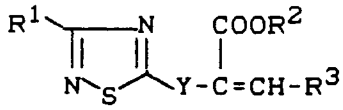 (I)

| Bsp. Nr. | $R^1$ | $R^2$ | $R^3$ | Y | Physikal. Konstante |
|---|---|---|---|---|---|
| 3 | 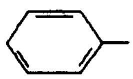 | $CH_3$ | $OCH_3$ | S | $^1H$-NMR*) ($CDCl_3$, 200 MHz) δ=3,80 (S, 3H), 4,07 (S, 3H), 7,35-7,5 (m, 3H) 8,15-8,25 (m, 2H) 8,10 (s, 1H) |
| 4 | $C(CH_3)_3$ | $CH_3$ | $N(CH_3)_2$ | S | Kp: 173-186° C 0.5 mbar |
| 5 | $n-C_3H_7$ | $CH_3$ | $N(CH_3)_2$ | S | $^1H$-NMR*) ($CDCl_3$, 200 MHz) δ=3,2-3,35 (s, 6H) |
| 6 | $n-C_3H_7$ | $C_2H_5$ | $N(CH_3)_2$ | -N-<br>$CH_3$ | Kp: 151-155° C 0.1 mbar |
| 7 | $CCl_2F$ | $C_2H_5$ | $N(CH_3)_2$ | -N-<br>$CH_3$ | $^1H$-NMR*) ($CDCl_3$, 200 MHz) δ=2,9-3,1 (s, 6H) |
| 8 | $CCl_2F$ | $C_2H_5$ | $OCH_3$ | -N-<br>$CH_3$ | Fp: 89- 94° C |
| 9 | $CCl_2F$ | $CH_3$ | $OCH_3$ | -N-<br>$CH_3$ | Kp: 173-178° C 0.4 mbar |
| 10 | $CClF_2$ | $CH_3$ | $OCH_3$ | -N-<br>$CH_3$ | Kp: 147-150° C 0.2 mbar |

Fortsetzung Tab. 2:

| Bsp. Nr. | $R^1$ | $R^2$ | $R^3$ | Y | Physikal. Konstante |
|---|---|---|---|---|---|
| 11 | $CF_3$ | $CH_3$ | $OCH_3$ | $-N(CH_3)-$ | Kp: 133-137° C 0,3 mbar |
| 12 | $C(CH_3)_3$ | $CH_3$ | $OCH_3$ | $-N(CH_3)-$ | Kp: 146-151° C 0,4 mbar |
| 13 | 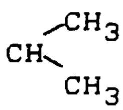 | $CH_3$ | $OCH_3$ | $-N(CH_3)-$ | Kp: 132-141° C 0,1 mbar |
| 14 | $C(CH_3)_3$ | $CH_3$ | $OCH_3$ | S | Kp: 115-121° C 0,1 mbar |
| 15 | $CCl_3$ | $CH_3$ | $OCH_3$ | $-N(CH_3)-$ | Kp: 114-121° C 0,7 mbar |
| 16 | 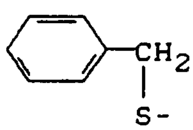 | CH3 | $OCH_3$ | $-N(CH_3)-$ | $^1H$-NMR*) ($CDCl_3$, 200 MHz) δ=7,2-7,4m, (3H) |
| 17 | $n-C_3H_7$ | $CH_3$ | $OCH_3$ | $-N(CH_3)-$ | Kp: 131-134° C 0,05 mbar |
| 18 | $CH_2Cl$ | $CH_3$ | $OCH_3$ | $-N(CH_3)-$ | $^1H$-NMR*) ($CDCl_3$, 200 MHz) δ=4,55 s, (2H) |
| 19 | $CCl_2F$ | $C_2H_5$ | $OC_2H_5$ | $-N(CH_3)-$ | $^1H$-NMR*) ($CDCl_3$, 200 MHz) δ=3,55 s, (3H) |

Fortsetzung Tab. 2:

| Bsp. Nr. | $R^1$ | $R^2$ | $R^3$ | Y | Physikal. Konstante |
|---|---|---|---|---|---|
| 20 | 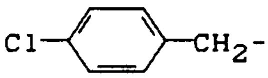 | $CH_3$ | $-OCH_3$ | $-N(CH_3)-$ | $^1H$-NMR* $\delta$=3,27s(3H,N-$CH_3$) 3,73s(3H, $CO_2CH_3$) 3,93s(3H, $COCH_3$) |
| 21 | 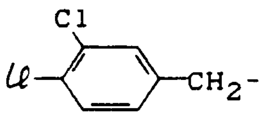 | $CH_3$ | $-OCH_3$ | $-N(CH_3)-$ | $^1H$-NMR* $\delta$=3,38s(3H,N-$CH_3$) 3,77s(3H, $CO_2CH_3$) 3,97s(3H, $COCH_3$) |
| 22 | 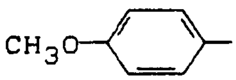 | $CH_3$ | $-OCH_3$ | $-N(CH_3)-$ | Fp.: 127° C |
| 23 | 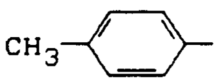 | $CH_3$ | $-OCH_3$ | $-N(CH_3)-$ | Fp.: 141° C |
| 24 | 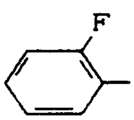 | $CH_3$ | $-OCH_3$ | $-N(CH_3)-$ | $^1H$-NMR* $\delta$=3,27s(3H,N-$CH_3$) 3,73s(3H,$CO_2CH_3$) 3,92s(3H,$COCH_3$) (Z-Isomer) |
| 25 | 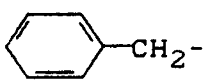 | $CH_3$ | $-OCH_3$ | $-N(CH_3)-$ | $^1H$-NMR* $\delta$=3,27s(3H,N-$CH_3$) 3,72s(3H,$CO_2CH_3$) 3,91s(3H,$OCH_3$) (Z-Isomer) |
| 26 | 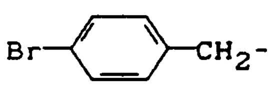 | $CH_3$ | $-OCH_3$ | $-N(CH_3)-$ | $^1H$-NMR* $\delta$=3,27s(3H,N-$CH_3$) =3,73s(34,$CO_2CH_3$) 3,92s(3H,$OCH_3$) (Z-Isomer) |
| 27 | $ClCH_2-$ | $CH_3$ | $-OCH_3$ | $-N(CH_3)-$ | $^1H$-NMR* $\delta$=3,31s(3H,N-$CH_3$) 3,76s(3H,$CO_2CH_3$) 3,96s(3H,$OCH_3$) (Z-Isomer) |

Fortsetzung Tab. 2:

| Bsp. Nr. | $R^1$ | $R^2$ | $R^3$ | Y | Physikal. Konstante |
|---|---|---|---|---|---|
| 28 | 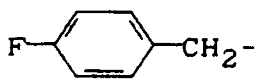 | $CH_3$ | $-OCH_3$ | $-N(CH_3)-$ | $^1$H-NMR* $\delta$=3,27s(3H,N-$CH_3$) 3,73s(3H,$CO_2CH_3$) 3,92s(3H,$OCH_3$) (Z-Isomer) |
| 29 | 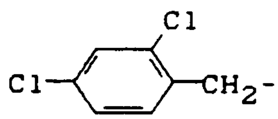 | $CH_3$ | $-OCH_3$ | $-N(CH_3)-$ | GC/MS:m/e=388 Ret. Index 2689 (Z-Isomeres) |
| 30 | 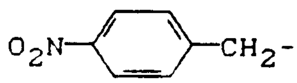 | $CH_3$ | $-OCH_3$ | $-N(CH_3)-$ | GC/MS:m/e=364 Ret. Index 2789 (Z-Isomeres) |
| 31 | 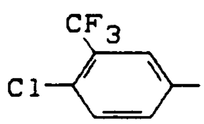 | $CH_3$ | $-OCH_3$ | $-N(CH_3)-$ | $^1$H-NMR* $\delta$=3,39s(3H,N-$CH_3$) 3,78s(3H,$CO_2CH_3$) 3,98s(3H,$OCH_3$) (Z-Isomer) |
| 32 | 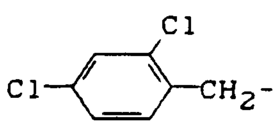 | $CH_3$ | $-OCH_3$ | $-N(CH_3)-$ | GC/MS:m/e=388 Ret. Index 2737 (E-Isomer) |
| 33 | 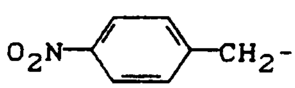 | $CH_3$ | $-OCH_3$ | $-N(CH_3)-$ | GC/MS:m/e=364 Ret. Index 2799 (E-Isomer) |
| 34 | 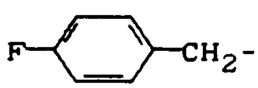 | $CH_3$ | $-OCH_3$ | $-N(CH_3)-$ | $^1$H-NMR* $\delta$=3,30s(3H,N-$CH_3$) 3,75s(3H,$CO_2CH_3$) 3,97s(3H,$OCH_3$) (E-Isomer) |
| 35 | $ClCH_2-$ | $CH_3$ | $-OCH_3$ | $-N(CH_3)-$ | Fp.: 91-93° C (E-Isomer) |
| 36 | 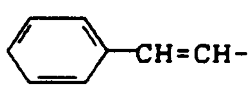 | $CH_3$ | $-OCH_3$ | $-N(CH_3)-$ | Fp.: 102-104° C (Z-Isomer) |

Fortsetzung Tab. 2:

| Bsp. Nr. | $R^1$ | $R^2$ | $R^3$ | Y | Physikal. Konstante |
|---|---|---|---|---|---|
| 37 | 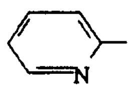 | $CH_3$ | $-OCH_3$ | $-N(CH_3)-$ | $^1$H-NMR* δ=3,43s(3H,N-$CH_3$) 3,76s(3H,$CO_2CH_3$) 3,96s(3H,$OCH_3$) (Z-Isomer) |
| 38 | 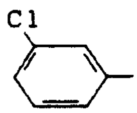 | $CH_3$ | $-OCH_3$ | $-N(CH_3)-$ | Fp.: 67° C |
| 39 | 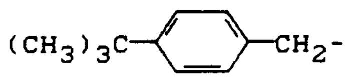 | $CH_3$ | $-OCH_3$ | $-N(CH_3)-$ | $^1$H-NMR* δ=3,27s(3H,N-$CH_3$) 3,71s(3H,$CO_2CH_3$) 3,88s(3H,$OCH_3$) |
| 40 | 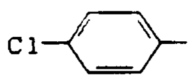 | $CH_3$ | $-OCH_3$ | $-N(CH_3)-$ | Fp. 103-106° C (Z-Isomer) |
| 41 | 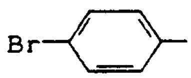 | $CH_3$ | $-OCH_3$ | $-N(CH_3)-$ | Fp. 90-91° C (Z-Isomer) |
| 42 | 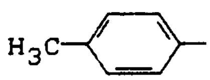 | $CH_3$ | $-OCH_3$ | $-N(CH_3)-$ | $^1$H-NMR* δ=3,27s(3H,N-$CH_3$) 3,72s(3H,$CO_2CH_3$) 3,91s(3H,$OCH_3$) (Z-Isomer) |
| 43 | 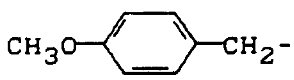 | $CH_3$ | $-OCH_3$ | $-N(CH_3)-$ | $^1$H-NMR* δ=3,37s(3H,N-$CH_3$) 3,76s(3H,$CO_2CH_3$) 3,95s(3H,$OCH_3$) (Z-Isomer) |
| 44 | 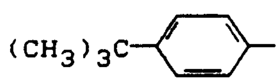 | $CH_3$ | $-OCH_3$ | $-N(CH_3)-$ | $^1$H-NMR* δ=3,38s(3H,N-$CH_3$) 3,75(3H,$CO_2CH_3$) 3,94s(3H,$OCH_3$) (Z-Isomer) |
| 45 | 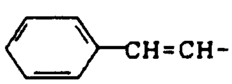 | $CH_3$ | $-OCH_3$ | $-N(CH_3)-$ | GC/MS:m/e=331 Ret. Index 2753 (E-Isomer) |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen

Beispiel (IV-1)

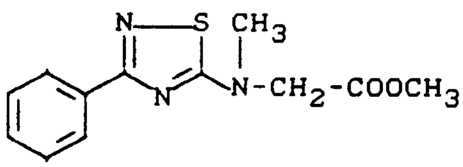

24 g (0.12 Mol) 5-Chlor-3-phenyl-1,2,4-thiadiazol, 16.8 g (0.12 Mol) N-Methylaminoessigsäuremethylester und 33.2 g (0.24 Mol) gemahlenes Kaliumcarbonat werden über Nacht in 200 ml trockenem 1.4-Dioxan erhitzt. Zur Aufarbeitung gießt man das Reaktionsgemisch auf Eiswasser und extrahiert dreimal mit Essigester. Nach Trocknen über Natriumsulfat erhält man nach Abdestillieren des Lösungsmittels und Umkristallisieren aus Ether/Ethanol 14.2 g (45% der Theorie) N-Methyl-N-(3-phenyl-1,2,4-thiadiazol-5-yl)-2-aminoessigsäuremethylester vom Schmelzpunkt 92 °C.

In analoger Weise zu der in dem Beispiel (IV-1) beschriebenen Methode und unter Berücksichtigung der Angaben in den Beschreibungen zu den erfindungsgemäßen Verfahren, werden die in der nachfolgenden Tabelle 3 aufgeführten Produkte der Formel (IV) erhalten.

Tabelle 3:

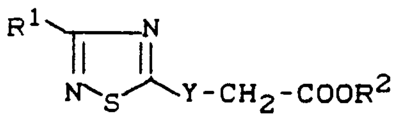

(IV)

| Bsp. | $R^1$ | $R^2$ | Y | Physikal. Daten |
|---|---|---|---|---|
| (IV-2) | 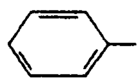 | $CH_3$ | S | $^1$H-NMR*) (DMSO, 200 MHz) δ=3,72 (s, 3H); 4,38 (s,2H);7,54 (mc,3H);8,11(mc,2H) |
| (IV-3) | 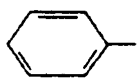 | H | S | Fp: 92° C |
| (IV-4) | $CFCl_2$ | $C_2H_5$ | -N-<br>$CH_3$ | $^1$H-NMR*) ($CDCl_3$, 200 MHz) δ=3,2 (s, 3H) |
| (IV-5) | $CFCl_2$ | $CH_3$ | -N-<br>$CH_3$ | $^1$H-NMR*) ($CDCl_3$, 200 MHz) δ=3,15 (s, 3H) |
| (IV-6) | $CFCl_2$ | $CH_3$ | S | Kp: 129-136° C/ 0,4 mbar |
| (IV-7) | $CFCl_2$ | $CH_3$ | S | Kp: 118-121° C/ 0,8 mbar |
| (IV-8) | $t-C_4H_9$ | $CH_3$ | S | Kp: 88-96° C/ 0,7 mbar |
| (IV-9) | $i-C_3H_7$ | $CH_3$ | S | $^1$H-NMR*) ($CDCl_3$, 200 MHz) δ=4,1 (s, 2H) |

| Bsp. | $R^1$ | $R^2$ | Y | Physikal. Daten |
|---|---|---|---|---|
| (IV-10) | $CF_2Cl$ | $CH_3$ | -N-<br>$CH_3$ | Kp: 119-124° C<br>0,2 mbar |
| (IV-11) | $CF_3$ | $CH_3$ | -N-<br>$CH_3$ | $^1H$-NMR($CDCl_3$, 200 MHz)<br>δ=3,2 (s, 3H) |
| (IV-12) | t-$C_4H_9$ | $CH_3$ | -N-<br>$CH_3$ | Kp: 108-112° C/<br>0,2 mbar |
| (IV-13) | i-$C_3H_7$ | $CH_3$ | -N-<br>$CH_3$ | Kp: 112-118° C/<br>0,2 mbar |
| (IV-17) | 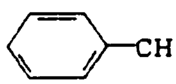 $-CH_2S-$ | $CH_3$ | -N-<br>$CH_3$ | $^1H$-NMR($CDCl_3$, 200 MHz)<br>δ=3,1 (s, 3H);<br>4,4 (s, 2H) |
| (IV-14) | $C_3H_7$ | $CH_3$ | S | $^1H$-NMR($CDCl_3$, 200 MHz)<br>δ=4,1 (s, 2H) |
| (IV-15) | $C_3H_7$ | $CH_3$ | -N-<br>$CH_3$ | $^1H$-NMR($CDCl_3$, 200 MHz)<br>δ=3,15 (s, 3H) |
| (IV-16) | $CHCl_2$ | $CH_3$ | -N-<br>$CH_3$ | $^1H$-NMR($CDCl_3$, 200 MHz)<br>δ=3,15 (s, 3H) |

| Bsp. | $R^1$ | $R^2$ | Y | Physikal. Daten |
|---|---|---|---|---|
| (IV-17) | 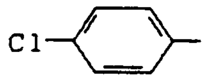 | $CH_3$ | $-N(CH_3)-$ | Fp.: 122° C |
| (IV-18) | 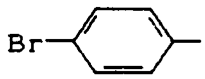 | $CH_3$ | $-N(CH_3)-$ | Öl |
| (IV-19) | 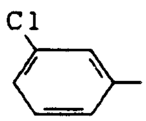 | $CH_3$ | $-N(CH_3)-$ | Fp.: 99° C |
| (IV-20) | 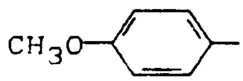 | $CH_3$ | $-N(CH_3)-$ | Öl |
| (IV-21) | 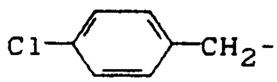 | $CH_3$ | $-N(CH_3)-$ | Öl |
| (IV-22) | 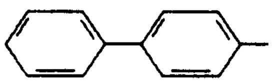 | H | $-N(CH_3)-$ | Fp.: 138° C |
| (IV-23) | 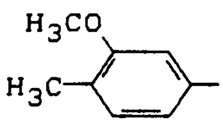 | $CH_3$ | $-N(CH_3)-$ | Fp.: 122° C |
| (IV-24) | 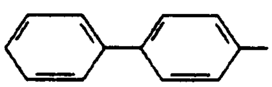 | $CH_3$ | $-N(CH_3)-$ | Fp.: 125° C |
| (IV-25) | 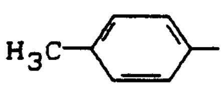 | H | $-N(CH_3)-$ | Fp.: 174° C |
| (IV-26) | 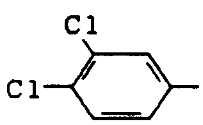 | $CH_3$ | $-N(CH_3)-$ | Fp.: 123° C |

| Bsp. | $R^1$ | $R^2$ | Y | Physikal. Daten |
|---|---|---|---|---|
| (IV-27) | 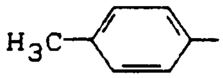 | $CH_3$ | $-N(CH_3)-$ | Fp.: 102° C |
| (IV-28) | 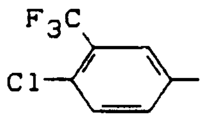 | $CH_3$ | $-N(CH_3)-$ | Fp.: 134° C |
| (IV-29) | 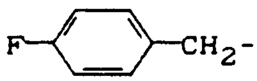 | $CH_3$ | $-N(CH_3)-$ | Fp.: 76-78° C |
| (IV-30) | 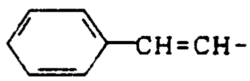 | H | $-N(CH_3)-$ | Fp.: 174-177° C |
| (IV-31) | $ClH_2C-$ | $CH_3$ | $-N(CH_3)-$ | Fp.: 60-62 °C |
| (IV-32) | 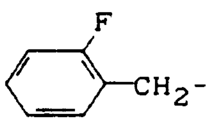 | $CH_3$ | $-N(CH_3)-$ | Fp.: 74-76° C |
| (IV-33) | 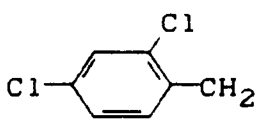 | $CH_3$ | $-N(CH_3)-$ | Fp.: 53-55° C |
| (IV-34) | 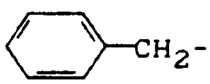 | $CH_3$ | $-N(CH_3)-$ | Fp.: 56-55° C |
| (IV-35) | 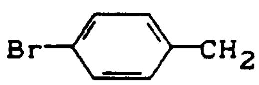 | $CH_3$ | $-N(CH_3)-$ | Fp.: 78-81° C |
| (IV-36) | 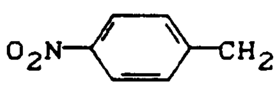 | $CH_3$ | $-N(CH_3)-$ | Fp.: 76° C |
| (IV-37) | 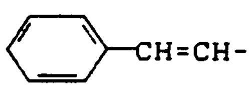 | $CH_3$ | $-N(CH_3)-$ | Fp.: 109-110° C |

| Bsp. | $R^1$ | $R^2$ | Y | Physikal. Daten |
|---|---|---|---|---|
| (IV-38) | 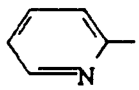 | $CH_3$ | $-N(CH_3)-$ | Fp.: 101-102° C |
| (IV-39) | 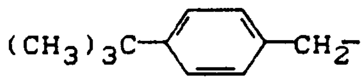 | $CH_3$ | $-N(CH_3)-$ | Öl |
| (IV-40) | 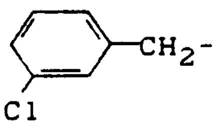 | $CH_3$ | $-N(CH_3)-$ | Öl |
| (IV-41) | 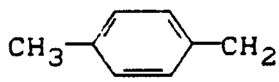 | $CH_3$ | $-N(CH_3)-$ | Fp.: 81° C |
| (IV-42) | 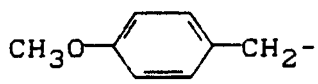 | $CH_3$ | $-N(CH_3)-$ | Fp.: 51-52° C |
| (IV-43) | 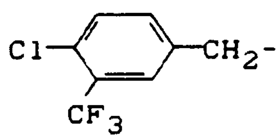 | $CH_3$ | $-N(CH_3)-$ | Fp.: 80° C |
| (IV-44) | 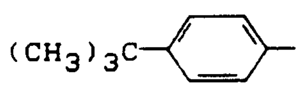 | $CH_3$ | $-N(CH_3)-$ | Fp.: 114° C |

*) Die $^1$H-NMR-Spektren wurden in Dimethylsulfoxid (DMSO oder $CDCl_3$) mit Tetramethylsilan (TMS) als innerem Standard aufgenommen. Angegeben ist die chemische Verschiebung als δ-Wert in ppm.

## Anwendungsbeispiele

In den folgenden Anwendungsbeispielen wurde die nachstehend aufgeführte Verbindung als Vergleichsubstanz eingesetzt:

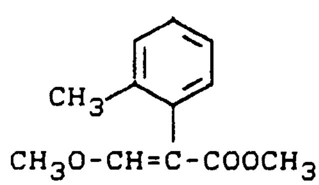

(A)

3-Methoxy-2-(2-methylphenyl)-acrylsäuremethylester
(bekannt aus EP 178 826).

Beispiel A

Pyricularia-Test (Reis)/protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit besprüht man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25 °C aufgestellt.

4 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 1.

Beispiel B

Leptosphaeria nodorum-Test (Weizen)/protektiv

Lösungsmittel: 100 Gewichtsteile Dimethylformamid
Emulgator: 0.25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20 °C und 100% relativer Luftfeuchtigkeit in einer Inkubationskabine,

Die Pflanzen werden in einem Gewächshaus bei einer Temperatur von ca. 15 °C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 1.

## Patentansprüche

**1.** Thiadiazol-substituierte Acrylsäureester der allgemeinen Formel (I),

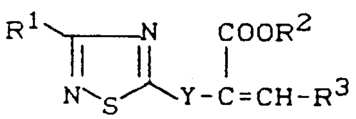

(I)

in welcher

$R^1$ für Wasserstoff, für unsubstituiertes oder ein- bis mehrfach, gleich oder verschieden substituiertes, geradkettiges oder verzweigtes Alkyl mit 1 bis 8 Kohlenstoffatomen steht, wobei folgende Substituenten genannt seien:
Fluor, Chlor, Brom, Jod, jeweils unsubstituiertes oder ein- bis fünffach, gleich oder verschieden substituiertes Arylthio und Aryloxy mit jeweils 6 bis 10 Kohlenstoffatomen im Arylteil, wobei als Arylsubstituenten die weiter unten in der Definition von $R^1$ aufgeführten Arylsubstituenten genannt seien;

$R^1$ weiterhin für geradkettiges oder verzweigtes Alkenyl mit 2 bis 8 Kohlenstoffatomen, für

jeweils unsubstituiertes oder im Arylteil einfach bis mehrfach, gleich oder verschieden substituiertes Aralkyl mit 1 bis 6 Kohlenstoffatomen im geradkettigen oder verzweigten Alkylteil, Aralkenyl mit 2 bis 6 Kohlenstoffatomen im geradkettigen oder verzweigten Alkenylteil oder für Aryl mit 6 bis 10 Kohlenstoffatomen im jeweiligen Arylteil steht, wobei als Arylsubstituenten jeweils infrage kommen:

Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, geradkettiges oder verzweigtes Alkoxycarbonyl mit 1 bis 4 Kohlenstoffatomen oder Alkoximinoalkyl mit jeweils 1 bis 6 Kohlenstoffatomen in den einzelnen Alkylteilen, Cycloalkyl mit 3 bis 8 Kohlenstoffatomen, zweifach verknüpftes Alkandiyl mit 3 bis 5 Kohlenstoffatomen, jeweils unsubstituiertes oder im Arylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach bis mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 8 Kohlenstoffatomen und 1 bis 4 gleichen oder verschiedenen Heteroatomen im Heteroarylteil und gegebenenfalls 1 bis 4 Kohlenstoffatomen im geradkettigen oder verzweigten Alkylteil;

$R^1$ außerdem für einen gegebenenfalls einfach bis mehrfach, gleich oder verschieden substituierten 5- oder 6-gliedrigen Heteroarylrest mit 1 bis 3 gleichen oder verschiedenen Heteroatomen steht, wobei als Substituenten die oben genannten Arylsubstituenten infrage kommen,

$R^2$ für geradkettiges oder verzweigtes Alkyl mit 1 bis 6 Kohlenstoffatomen steht,

$R^3$ für Dialkylamino mit jeweils 1 bis 6 Kohlenstoffatomen in den einzelnen geradkettigen oder verzweigten, gleichen oder verschiedenen Alkylteilen steht oder für einen Rest -Z-$R^4$ steht,

Y für Sauerstoff, Schwefel oder für einen Rest

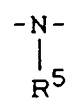

steht,

$R^4$ für geradkettiges oder verzweigtes Alkyl mit 1 bis 6 Kohlenstoffatomen oder für unsubstituiertes oder im Arylteil einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten die bei $R^1$ genannten infrage kommen,

$R^5$ für Wasserstoff oder für geradkettiges oder verzweigtes Alkyl mit 1 bis 6 Kohlenstoffatomen steht und

Z für Sauerstoff oder Schwefel steht,

deren Isomere und Isomerengemische.

**2.** Thiadiazol-substituierte Acrylsäureester der Formel (I) gemäß Anspruch 1, wobei

$R^1$ für Wasserstoff, Methyl, Ethyl, n- oder i-Propyl, n-, i-, s- oder t-Butyl, ein- bis sechsfach, gleich oder verschieden durch Fluor und Chlor substituiertes geradkettiges oder verzweigtes Alkyl mit 1 bis 4 Kohlenstoffatomen steht oder für geradkettiges oder verzweigtes Alkyl mit 1 bis 4 Kohlenstoffatomen steht, welches durch jeweils unsubstituiertes oder ein- bis dreifach, gleich oder verschieden substituiertes Phenylthio oder Phenyloxy substituiert ist, wobei als Phenylsubstituenten die weiter unten in der Definition von $R^1$ aufgeführten Substituenten genannt seien;

$R^1$ für Allyl, n- oder i-Butenyl, für jeweils gegebenenfalls im Arylteil oder im Heteroarylteil ein- bis dreifach, gleich oder verschieden substituiertes Benzyl, Phenylethyl, Phenylethenyl, Phenyl, Naphthyl, Pyridyl, Thienyl oder Furyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, But-2-en-di-1,4-yl, 1,4-Butandiyl oder jeweils unsubstituiertes oder im Phenylteil ein-bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und Trifluormethylthio substituiertes Phenyl, Benzyl, Phenoxy oder Benzyloxy,

$R^2$ für Methyl, Ethyl, n- oder i-Propyl, n-, i-, s- oder t-Butyl steht,

$R^3$ für Dialkylamino mit jeweils 1 bis 4 Kohlenstoffatomen in den einzelnen geradkettigen oder verzweigten, gleichen oder verschiedenen Alkylteilen steht oder für einen Rest -Z-$R^4$ steht,

Y für Sauerstoff, Schwefel oder für einen Rest

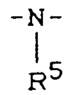

steht,

$R^4$ für Methyl, Ethyl, n- oder i-Propyl, n-, i-, s- oder t-Butyl oder für unsubstituiertes oder ein- bis dreifach, gleich oder verschieden substituiertes Benzyl steht, wobei als Substituenten die bei $R^1$ genannten infrage kommen;

$R^5$ für Wasserstoff, Methyl, Ethyl, n- oder i-Propyl, n-, i-, s- oder t-Butyl und

Z für Sauerstoff oder Schwefel stehen.

**3.** Thiadiazol-substituierte Acrylsäureester der Formel (I) gemaß Anspruch 1, wobei

$R^1$ für Wasserstoff, Methyl, Ethyl, n- oder i-Propyl, t-Butyl, ein- bis fünffach, gleich oder verschieden durch Fluor und Chlor substituiertes Alkyl mit 1 oder 2 Kohlenstoffatomen oder für Alkyl mit 1 oder 2 Kohlenstoffatomen steht, welches durch jeweils unsubstituiertes oder ein- oder zweifach, gleich oder verschieden substituiertes Phenylthio und Phenyloxy substituiert ist, wobei als Phenylsubstituenten die weiter unten in der Definition von $R^1$ aufgeführten Substituenten genannt seien oder

$R^1$ für jeweils gegebenenfalls ein- bis dreifach, gleich oder verschieden substituiertes Phenyl oder Naphthyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls ein- oder zweifach, gleich oder verschieden durch Fluor, Chlor, Brom, Methyl und Ethyl substituiertes Phenyl, Phenoxy, Benzyl oder Benzyloxy,

$R^2$ für Methyl oder Ethyl steht,

$R^3$ für Dimethylamino, Diethylamino oder für einen Rest -Z-$R^4$ steht,

Y für Sauerstoff, Schwefel oder für einen Rest

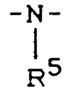

steht,

$R^4$ für Methyl, Ethyl, n- oder i-Propyl oder Benzyl steht,

$R^5$ für Wasserstoff, Methyl oder Ethyl steht und

Z für Sauerstoff oder Schwefel steht,

**4.** Thiadiazol-substituierte Acrylsäureester der Formel (I) gemäß Anspruch 1, wobei

$R^1$ für Wasserstoff, Methyl, Ethyl, n- oder i-Propyl, t-Butyl, ein- bis dreifach, gleich oder verschieden durch Fluor und Chlor substituiertes Methyl oder für jeweils gegebenenfalls einfach oder zweifach, gleich oder verschieden substituiertes Phenyl, Phenyloxymethyl oder

Phenylthiomethyl steht, wobei als Substituenten infrage kommen:
Fluor, Chlor, Brom, Cyano, Nitro, Methyl, Ethyl, Methoxy, Ethoxy, Methylthio, Trifluormethyl, Trifluormethoxy, Trifluormethylthio, Methoxycarbonyl, Ethoxycarbonyl, Methoximinomethyl, Cyclopentyl, 1,3-Propandiyl, Methoximinoethyl oder gegebenenfalls ein- oder zweifach, gleich oder verschieden durch Fluor, Chlor, Brom oder Methyl substituiertes Phenyl, Phenoxy, Benzyl oder Benzyloxy,

$R^2$ für Methyl oder Ethyl steht,

$R^3$ für Methoxy, Ethoxy, Methylthio oder Dimethylamino steht und

Y für einen N-Methyl-Rest oder Schwefel steht.

**5.** Verfahren zur Herstellung von Thiadiazol-substituierten Acrylsäureestern der allgemeinen Formel (I),

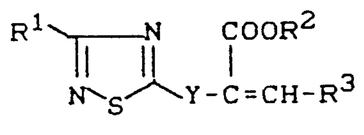

(I)

in welcher

$R^1$, $R^2$ und $R^3$ die in Anspruch 1 angegebene Bedeutung haben,

dadurch gekennzeichnet, daß man

(a) Thiadiazol-substituierte Acrylsäureester der Formel (Ia),

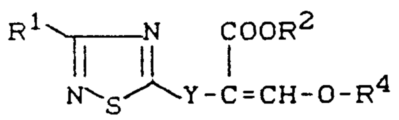

(Ia)

in welcher

$R^1$, $R^2$, $R^4$ und Y die oben angegebene Bedeutung haben, erhält,

indem man Hydroxyacrylsäureester oder deren Alkalimetallsalze der Formel (II),

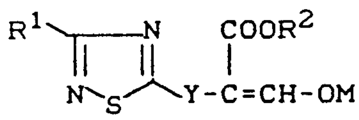

(II)

in welcher

M für Wasserstoff oder für ein Alkalimetallkation steht und

$R^1$, $R^2$ und Y die oben angegebene Bedeutung haben,

mit Alkylierungsmitteln der Formel (III),

$R^4$-$E^1$ (III)

in welcher

$E^1$ für eine elektronenanziehende Abgangsgruppe steht und

$R^4$ die oben angegebene Bedeutung hat,

gegebenenfalls in Gegenwart eines Verdünnungsmittels und gegebenenfalls in Gegenwart eines Reaktionshilfsmittels umsetzt oder

(b) Thiadiazol-substituierte Acrylsäureester der Formel (Ib),

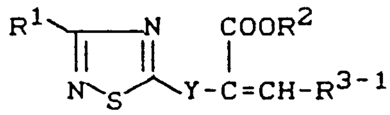

(Ib)

in welcher
$R^{3-1}$ für Dialkylamino steht und
$R^1$, $R^2$ und Y die oben angegebene Bedeutung haben, erhält,
indem man substituierte Essigsäureester der Formel (IV),

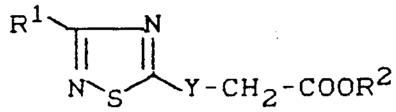

(IV)

in welcher
$R^1$, $R^2$ und Y die oben angegebene Bedeutung haben,
mit Formamid-Derivaten der Formel (V),

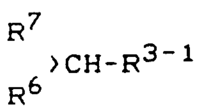

(V)

in welcher
$R^6$ und $R^7$ unabhängig voneinander jeweils für Alkoxy oder Dialkylamino stehen und
$R^{3-1}$ die oben angegebene Bedeutung hat,
gegebenenfalls in Gegenwart eines Verdünnungsmittels umsetzt oder
(c) Thiadiazol-substituierte Acrylsäureester der Formel (Ic),

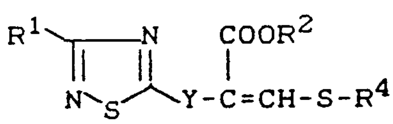

(Ic)

in welcher
$R^1$, $R^2$, $R^4$ und Y die oben angegebene Bedeutung haben, erhält,
indem man substituierte Acrylsäureester der Formel (VI),

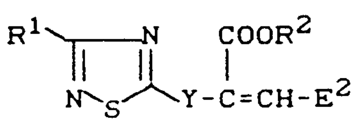

(VI)

in welcher
$E^2$ für eine elektronenanziehende Abgangsgruppe steht und
$R^1$, $R^2$ und Y die oben angegebene Bedeutung haben,
mit Thiolen der Formel (VII)

$R^4$-SH (VII)

in welcher
$R^4$ die oben angegebene Bedeutung hat,
gegebenenfalls in Gegenwart eines Verdünnungsmittels und gegebenenfalls in Gegenwart eines Reaktionshilfsmittels umsetzt.

**6.** Fungizide Mittel, gekennzeichnet durch einen Gehalt an mindestens einem Thiadiazol-substituierten Acrylsäureester der Formel (I) gemäß den Ansprüchen 1 bis 5.

**7.** Verfahren zur Bekämpfung von Pilzen, dadurch gekennzeichnet, daß man Thiadiazol-substituierte Acrylsäureester der Formel (I) gemäß den Ansprüchen 1 bis 6 auf die Pilze und/oder ihren Lebensraum einwirken läßt.

**8.** Verwendung von Thiadiazol-substituierten Acrylsäureestern der Formel (I) gemäß den Ansprüchen 1 bis 6 zur Bekämpfung von Pilzen.

**9.** Verfahren zur Herstellung von fungiziden Mitteln, dadurch gekennzeichnet, daß man Thiadiazol-substituierte Acrylsäureester der Formel (I) gemäß den Ansprüchen 1 bis 6 mit Streckmitteln und/oder oberflächenaktiven Substanzen vermischt.

**10.** Hydroxyacrylsäureester der Formel (II)

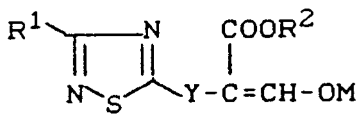

(II)

in welcher
$R^1$ und $R^2$ die in Anspruch 1 angegebene Bedeutung haben und
M für Wasserstoff oder für ein Alkalimetallkation steht.

**11.** Verfahren zur herstellung von Hydroxyacrylsäureestern der Formel (II) gemäß Anspruch 11, dadurch gekennzeichnet, daß man Thiadiazol-substituierte Essigsäureester der Formel (IV)

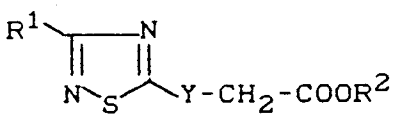

(IV)

in welcher
$R^1$, $R^2$ und Y die in Anspruch 10 angegebene Bedeutung haben,
mit Ameisensäureestern der Formel (VIII),

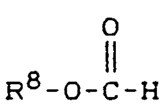

(VIII)

in welcher
$R^8$ für Alkyl steht,
gegebenenfalls in Gegenwart eines Verdünnungsmittels und gegebenenfalls in Gegenwart eines basischen Reaktionshilfsmittels bei Temperaturen zwischen -20 °C und +50 °C umsetzt und gegebenenfalls anschließend mit einer Säure hydrolysiert.

**12.** Thiadiazol-substituierte Essigsäureester der Formel (IVa)

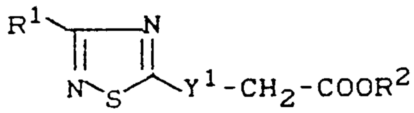

(IVa)

in welcher

$Y^1$ für Sauerstoff oder für einen N-Alkylrest steht,

$R^1$ und $R^2$ die in Anspruch 1 angegebene Bedeutung haben,

ausgenommen die Verbindung N-Methyl-N-[3-(trichlormethyl)-1,2,4-thiadiazol-5-yl]-glycinethylester.

**13.** Verfahren zur Herstellung von Thiadiazol-substituierten Essigsäureestern der Formel (IVa) gemäß Anspruch 13, dadurch gekennzeichnet, daß man 1,2,4-Thiadiazolderivate der Formel (IX),

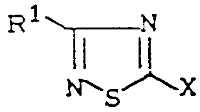

(IX)

in welcher

$R^1$ die in Anspruch 12 angegebene Bedeutung hat und

X für Halogen steht,

mit Essigsäureesterderivaten der Formel (X)

$HY^1\text{-}CH_2\text{-}COOR^2$ (X)

in welcher

$R^2$ und $Y^1$ die in Anspruch 12 angegebene Bedeutung haben oder deren Hydrochloride, gegebenenfalls in Gegenwart eines Verdünnungsmittels und gegebenenfalls in Gegenwart einer Base umsetzt.

**14.** Substituierte Acrylsäureester der Formel (VI)

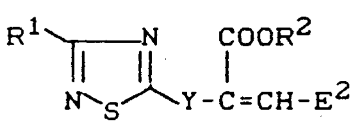

(VI)

in welcher

$R^1$ und $R^2$ die in Anspruch 1 angegebene Bedeutung haben,

Y für Sauerstoff, Schwefel oder für einen Rest

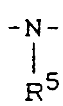

steht, wobei

$R^5$ für Wasserstoff oder Alkyl steht und

$E^2$ für eine elektronenanziehende Abgangsgruppe steht,

**15.** Verfahren zur Herstellung von substituierten Acrylsäureestern der Formel (VI) gemäß Anspruch 15, dadurch gekennzeichnet, daß man Hydroxyacrylsäureester der Formel (II)

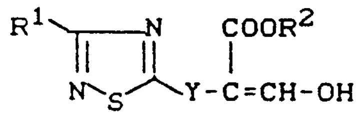

(II)

in welcher
$R^1$ $R^2$ und Y die in Anspruch 14 angegebene Bedeutung haben,
mit Säurechloriden der Formel (XI),

$R^9$-Cl (XI)

in welcher
$R^9$ für einen Acyl- oder Sulfonylrest steht,
gegebenenfalls in Gegenwart eines Verdünnungsmittels und gegebenenfalls in Gegenwart eines Säurebindemittels bei Temperaturen zwischen -20 °C und +120 °C umsetzt,

**16.** Verwendung von Verbindungen der Formeln (II), (IVa) und (VI) gemäß den Ansprüchen 10 bis 15 als Zwischenprodukte.

## Claims

**1.** Thiadiazole-substituted acrylic acid esters of the general formula (I)

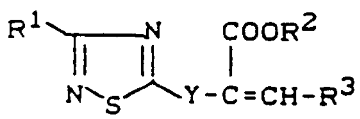

(I)

in which
$R^1$ represents hydrogen, or straight-chain or branched alkyl having 1 to 8 carbon atoms which is unsubstituted or monosubstituted to polysubstituted by identical or different substituents, suitable substituents being the following:
fluorine, chlorine, bromine, iodine, or arylthio and aryloxy each having 6 to 10 carbon atoms in the aryl moiety, and in each case unsubstituted or monosubstituted to pentasubstituted by identical or different substituents, where the aryl substituents mentioned below in the definition of $R^1$ may be mentioned as aryl substituents;
$R^1$ furthermore represents straight-chain or branched alkenyl having 2 to 8 carbon atoms, aralkyl having 1 to 6 carbon atoms in the straight-chain or branched alkyl moiety, in each case unsubstituted or monosubstituted to polysubstituted in the aryl moiety by identical or different substituents, aralkenyl having 2 to 6 carbon atoms in the straight-chain or branched alkenyl moiety or aryl having 6 to 10 carbon atoms in the respective aryl moiety, suitable aryl substituents in each case being:
halogen, cyano, nitro, in each case straight-chain or branched alkyl, alkoxy or alkylthio each having 1 to 6 carbon atoms, in each case straight-chain or branched halogenoalkyl, halogenoalkoxy or halogenoalkylthio each having 1 to 4 carbon atoms and 1 to 9 identical or different halogen atoms, straight-chain or branched alkoxycarbonyl having 1 to 4 carbon atoms or alkoximinoalkyl each having 1 to 6 carbon atoms in the individual alkyl moieties, cycloalkyl having 3 to 8 carbon atoms, doubly linked alkanediyl having 3 to 5 carbon atoms, aryl, aralkyl, aryloxy or aralkyloxy each having 6 to 10 carbon atoms in the aryl moiety and optionally 1 to 4 carbon atoms in the straight-chain or branched alkyl moiety and in each case unsubstituted or monosubstituted to polysubstituted in the aryl moiety by identical or different substituents from the series comprising halogen, alkyl, alkoxy, alkylthio, halogenoalkyl, halogenoalkoxy or halogenoalkylthio each having 1 to 4 carbon atoms and optionally 1 to 9 identical or different halogen atoms, or heteroarylalkyl or heteroaryl each having 2 to 8 carbon atoms and 1 to 4 identical or different heteroatoms, in the heteroaryl moiety and optionally 1 to 4 carbon atoms in the straight-chain or branched alkyl moiety and in each

case optionally monosubstituted to polysubstituted in the heteroaryl moiety by identical or different substituents from the series comprising halogen, alkyl, alkoxy, alkylthio, halogenoalkyl, halogenoalkoxy or halogenoalkylthio each having 1 to 4 carbon atoms and optionally 1 to 9 identical or different halogen atoms;

$R^1$ additionally represents a 5- or 6-membered heteroaryl radical having 1 to 3 identical or different heteroatoms and optionally monosubstituted to polysubstituted by identical or different substituents, suitable substituents being the abovementioned aryl substituents,

$R^2$ represents straight-chain or branched alkyl having 1 to 6 carbon atoms,

$R^3$ represents dialkylamino each having 1 to 6 carbon atoms in the individual straight-chain or branched, identical or different alkyl moieties or represents a radical $-Z-R^4$,

Y represents oxygen, sulphur or a radical

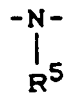

$R^4$ represents straight-chain or branched alkyl having 1 to 6 carbon atoms or aralkyl having 1 to 4 carbon atoms in the straight-chain or branched alkyl moiety and 6 to 10 carbon atoms in the aryl moiety and unsubstituted or monosubstituted to polysubstituted in the aryl moiety by identical or different substituents, suitable aryl substituents being those mentioned for $R^1$,

$R^5$ represents hydrogen or straight-chain or branched alkyl having 1 to 6 carbon atoms and

Z represents oxygen or sulphur, their isomers and isomer mixtures.

**2.** Thiadiazole-substituted acrylic acid esters of the formula (I) according to Claim 1, where

$R^1$ represents hydrogen, methyl, ethyl, n- or i-propyl, n-, i-, s- or t-butyl, straight-chain or branched alkyl having 1 to 4 carbon atoms, which is monosubstituted to hexasubstituted by identical or different substituents from the series comprising fluorine and chlorine, or straight-chain or branched alkyl having 1 to 4 carbon atoms which is substituted by phenylthio or phenyloxy which in each case are unsubstituted or monosubstituted to trisubstituted by identical or different substituents, suitable phenyl substituents which may be mentioned being the substituents mentioned below in the definition of $R^1$;

$R^1$ represents allyl, n- or i-butenyl, or benzyl, phenylethyl, phenylethenyl, phenyl, naphthyl, pyridyl, thienyl or furyl in each case optionally monosubstituted to trisubstituted in the aryl moiety or in the heteroaryl moiety by identical or different substituents, suitable substituents in each case being:
fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl, but-2-ene-di-1,4-yl, 1,4-butanediyl, or phenyl, benzyl, phenoxy or benzyloxy in each case unsubstituted or monosubstituted to trisubstituted in the phenyl moiety by identical or different substituents from the series comprising fluorine, chlorine, bromine, methyl, ethyl, methoxy, ethoxy, methylthio, trifluoromethyl, difluoromethoxy, trifluoromethoxy and trifluoromethylthio,

$R^2$ represents methyl, ethyl, n- or i-propyl, n-, i-, s-or t-butyl,

$R^3$ represents dialkylamino each having 1 to 4 carbon atoms in the individual straight-chain or branched, identical or different alkyl moieties or a radical $-Z-R^4$,

Y represents oxygen, sulphur or a radical

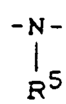

$R^4$ represents methyl, ethyl, n- or i-propyl, n-, i-, s- or t-butyl or benzyl which is unsubstituted or

monosubstituted to trisubstituted by identical or different substituents, suitable substituents being those mentioned for $R^1$;

$R^5$ represents hydrogen, methyl, ethyl, n- or i-propyl, n-, i-, s- or t-butyl and

Z represents oxygen or sulphur.

**3.** Thiadiazole-substituted acrylic acid esters of the formula (I) according to Claim 1, where

$R^1$ represents hydrogen, methyl, ethyl, n- or i-propyl, t-butyl, alkyl having 1 or 2 carbon atoms, which is monosubstituted to pentasubstituted by identical or different substituents from the series comprising fluorine and chlorine or alkyl having 1 or 2 carbon atoms, which is substituted by phenylthio and phenyloxy which are in each case unsubstituted or monosubstituted or disubstituted by identical or different substituents, suitable phenyl substituents which may be mentioned being those mentioned below in the definition of $R^1$ or

$R^1$ represents phenyl or naphthyl in each case optionally monosubstituted to trisubstituted by identical or different substituents, suitable substituents in each case being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl, cyclohexyl, 1,3-propanediyl, 1,4-butanediyl or phenyl, phenoxy, benzyl or benzyloxy in each case optionally monosubstituted or disubstituted by identical or different substituents from the series comprising fluorine, chlorine, bromine, methyl and ethyl,

$R^2$ represents methyl or ethyl,

$R^3$ represents dimethylamino, diethylamino or a radical -Z-$R^4$,

Y represents oxygen, sulphur or a radical

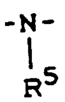

$R^4$ represents methyl, ethyl, n- or i-propyl or benzyl,

$R^5$ represents hydrogen, methyl or ethyl and

Z represents oxygen or sulphur.

**4.** Thiadiazole-substituted acrylic acid esters of the formula (I) according to Claim 1, where

$R^1$ represents hydrogen, methyl, ethyl, n- or i-propyl, t-butyl, methyl which is monosubstituted to trisubstituted by identical or different substituents from the series comprising fluorine and chlorine or phenyl, phenyloxymethyl or phenylthiomethyl which are in each case optionally monosubstituted or disubstituted by identical or different substituents, suitable substituents being:

fluorine, chlorine, bromine, cyano, nitro, methyl, ethyl, methoxy, ethoxy, methylthio, trifluoromethyl, trifluoromethoxy, trifluoromethylthio, methoxycarbonyl, ethoxycarbonyl, methoximinomethyl, cyclopentyl, 1,3-propanediyl, methoximinoethyl or phenyl, phenoxy, benzyl or benzyloxy which are optionally monosubstituted or disubstituted by identical or different substituents from the series comprising fluorine, chlorine, bromine or methyl,

$R^2$ represents methyl or ethyl,

$R^3$ represents methoxy, ethoxy, methylthio or dimethylamino and

Y represents an N-methyl radical or sulphur.

**5.** Process for the preparation of thiadiazole-substituted acrylic acid esters of the general formula (I)

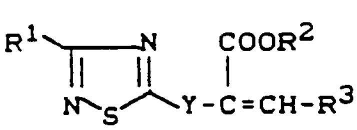

in which

$R^1$, $R^2$ and $R^3$ have the meaning indicated in Claim 1, characterized in that:

(a) thiadiazole-substituted acrylic acid esters of the formula (Ia)

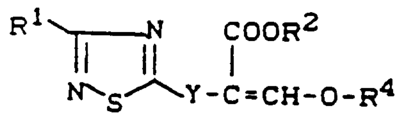

(Ia)

in which
$R^1$, $R^2$, $R^4$ and Y have the abovementioned meaning,
are obtained when hydroxyacrylic acid esters or their alkali metal salts of the formula (II)

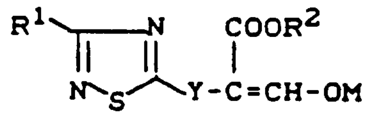

(II)

in which

| | |
|---|---|
| M | represents hydrogen or an alkali metal cation and |
| $R^1$, $R^2$ and Y | have the abovementioned meaning, |

are reacted with alkylating agents of the formula (III)

$R^4$-$E^1$ (III)

in which

| | |
|---|---|
| $E^1$ | represents an electron-withdrawing leaving group and |
| $R^4$ | has the abovementioned meaning, |

if appropriate in the presence of a diluent and if appropriate in the presence of a reaction aid, or
(b) thiadiazole-substituted acrylic acid esters of the formula (Ib)

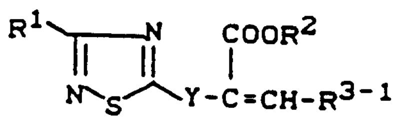

(Ib)

in which

| | |
|---|---|
| $R^{3-1}$ | represents dialkylamino and |
| $R^1$, $R^2$ and Y | have the abovementioned meaning, |

are obtained when substituted acetic acid esters of the formula (IV)

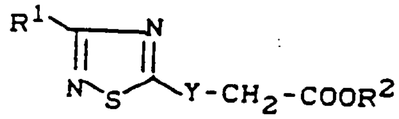

(IV)

in which
$R^1$, $R^2$ and Y have the abovementioned meaning,
are reacted with formamide derivatives of the formula (V)

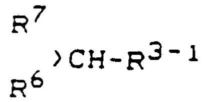 (V)

in which

$R^6$ and $R^7$ independently of one another in each case represent alkoxy or dialkylamino and

$R^{3-1}$ has the abovementioned meaning,

if appropriate in the presence of a diluent; or

(c) thiadiazole-substituted acrylic acid esters of the formula (Ic)

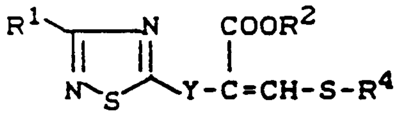 (Ic)

in which

$R^1$, $R^2$, $R^4$ and Y have the abovementioned meaning, are obtained when substituted acrylic acid esters of the formula (VI)

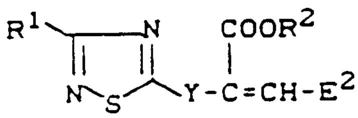 (VI)

in which

$E^2$ represents an electron-withdrawing leaving group and

$R^1$, $R^2$ and Y have the abovementioned meaning,

are reacted with thiols of the formula (VII)

$R^4$-SH (VII)

in which

$R^4$ has the abovementioned meaning,

if appropriate in the presence of a diluent and if appropriate in the presence of a reaction aid.

**6.** Fungicidal agents, characterized in that they contain at least one thiadiazole-substituted acrylic acid ester of the formula (I) according to Claims 1 to 5.

**7.** Method for combating fungi, characterized in that thiadiazole-substituted acrylic acid esters of the formula (I) according to Claims 1 to 5 are allowed to act on the fungi and/or their environment.

**8.** Use of thiadiazole-substituted acrylic acid esters of the formula (I) according to Claims 1 to 5 for combating fungi.

**9.** Process for the preparation of fungicidal agents, characterized in that thiadiazole-substituted acrylic acid esters of the formula (I) according to Claims 1 to 5 are mixed with extenders and/or surface-active substances.

**10.** Hydroxyacrylic acid esters of the formula (II)

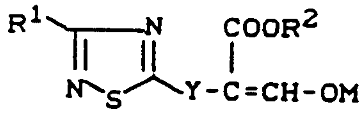

(II)

in which

| | |
|---|---|
| $R^1$ and $R^2$ | have the meaning indicated in Claim 1 and |
| M | represents hydrogen or an alkali metal cation. |

**11.** Process for the preparation of hydroxyacrylic acid esters of the formula (II) according to Claim 10, characterized in that thiadiazole-substituted acetic acid esters of the formula (IV)

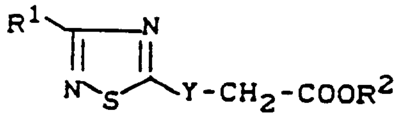

(IV)

in which
$R^1$, $R^2$ and Y have the meaning indicated in Claim 10,
are reacted with formic acid esters of the formula (VIII)

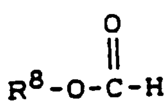

(VIII)

in which
$R^8$ represents alkyl,
if appropriate in the presence of a diluent and if appropriate in the presence of a basic reaction auxiliary at temperatures between -20 °C and +50 °C and, if appropriate, are then hydrolyzed with an acid.

**12.** Thiadiazole-substituted acetic acid esters of the formula (IVa)

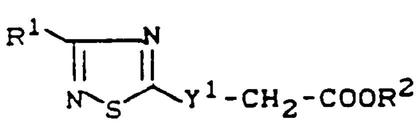

(IVa)

in which

| | |
|---|---|
| $Y^1$ | represents oxygen or an N-alkyl radical, |
| $R^1$ and $R^2$ | have the meaning indicated in Claim 1, |

excluding the compound N-methyl-N-[3-(trichloromethyl)-1,2,4-thiadiazol-5-yl]-glycine ethyl ester.

**13.** Process for the preparation of thiadiazole-substituted acetic acid esters of the formula (IVa) according to Claim 12, characterized in that 1,2,4-thiadiazole derivatives of the formula (IX)

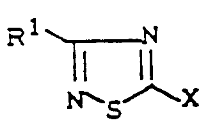

(IX)

in which
$R^1$ has the meaning indicated in Claim 12 and
X represents halogen,
are reacted with acetic acid ester derivatives of the formula (X)

$HY^1$-$CH_2$-$COOR^2$ (X)

in which
$R^2$ and $Y^1$ have the meaning indicated in Claim 12, or their hydrochlorides,
if appropriate in the presence of a diluent and if appropriate in the presence of a base.

**14.** Substituted acrylic acid esters of the formula (VI)

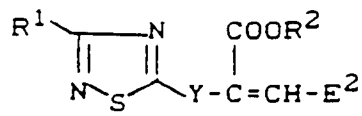

(VI)

in which
$R^1$ and $R^2$ have the meaning indicated in Claim 1,
Y represents oxygen, sulphur or a radical

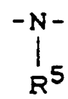

where
$R^5$ represents hydrogen or alkyl and
$E^2$ represents an electron-withdrawing leaving group.

**15.** Process for the preparation of substituted acrylic acid esters of the formula (VI) according to Claim 14, characterized in that hydroxyacrylic acid esters of the formula (II)

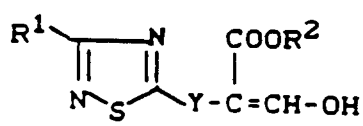

(II)

in which
$R^1$, $R^2$ and Y have the meaning indicated in Claim 14,
are reacted with acid chlorides of the formula (XI)

$R^9$-Cl (XI)

in which
$R^9$ represents an acyl or sulphonyl radical,
if appropriate in the presence of a diluent and if appropriate in the presence of an acid-binding agent at temperatures between -20 ° C and + 120 ° C.

**16.** Use of compounds of the formulae (II), (IVa) and (VI) according to Claims 10 to 15 as intermediates.

## Revendications

**1.** Esters d'acide acrylique à substituant thiadiazole de formule générale (I),

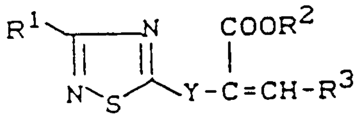

(I)

dans laquelle

$R^1$ représente l'hydrogène, un groupe alkyle linéaire ou ramifié ayant 1 à 8 atomes de carbone, non substitué ou portant un ou plusieurs substituants identiques ou différents parmi lesquels on mentionne les suivants : fluor, chlore, brome, iode, restes arylthio et aryloxy ayant chacun 6 à 10 atomes de carbone dans la partie aryle, non substitués ou portant chacun 1 à 5 substituants identiques ou différents, avec comme substituants de la partie aryle les substituants de cette partie indiqués dans la suite de la définition de $R^1$ ;

$R^1$ représente en outre un groupe alcényle linéaire ou ramifié ayant 2 à 8 atomes de carbone, un reste aralkyle de 1 à 6 atomes de carbone dans la partie alkyle linéaire ou ramifiée, un reste aralcényle de 2 à 6 atomes de carbone dans la partie alcényle linéaire ou ramifiée ou un reste aryle, avec 6 à 10 atomes de carbone dans chaque partie aryle, chacun étant non substitué ou portant dans la partie aryle un ou plusieurs substituants identiques ou différents, en considérant dans chaque cas comme substituants de la partie aryle : un halogène, un radical cyano, nitro, un radical alkyle, alkoxy ou alkylthio à chaîne droite ou ramifiée avec chacun 1 à 6 atomes de carbone, un radical halogénalkyle, halogénalkoxy ou halogénalkylthio à chaîne droite ou ramifiée avec chacun 1 à 4 atomes de carbone et 1 à 9 atomes d'halogènes identiques ou différents, un reste alkoxycarbonyle linéaire ou ramifié avec 1 à 4 atomes de carbone, ou alkoximinoalkyle avec 1 à 6 atomes de carbone dans les parties alkyle individuelles, un radical cycloalkyle ayant 3 à 8 atomes de carbone, un radical alcanediyle divalent ayant 3 à 5 atomes de carbone, un radical aryle, aralkyle, aryloxy ou aralkyloxy avec chacun 6 à 10 atomes de carbone dans la partie aryle et le cas échéant 1 à 4 atomes de carbone dans la partie alkyle linéaire ou ramifiée, chacun étant non substitué ou portant dans la partie aryle un à plusieurs substituants, identiques ou différents, halogéno, alkyle, alkoxy, alkylthio, halogénalkyle, halogénalkoxy ou halogénalkylthio avec chacun 1 à 4 atomes de carbone et le cas échéant 1 à 9 atomes d'halogènes identiques ou différents, ou un radical hétéroarylalkyle ou hétéroaryle avec chacun 2 à 8 atomes de carbone et 1 à 4 hétéro-atomes identiques ou différents dans la partie hétéroaryle et le cas échéant 1 à 4 atomes de carbone dans la partie alkyle linéaire ou ramifiée, chacun portant le cas échéant dans la partie hétéroaryle un à plusieurs substituants, identiques ou différents, halogéno, alkyle, alkoxy, alkylthio, halogénalkyle, halogénalkoxy ou halogénalkylthio avec chacun 1 à 4 atomes de carbone et le cas échéant 1 à 9 atomes d'halogènes identiques ou différents ;

$R^1$ représente en outre un reste hétéroaryle pentagonal ou hexagonal avec 1 à 3 hétéroatomes identiques ou différents, portant le cas échéant un à plusieurs substituants identiques ou différents, en considérant comme substituants, les substituants de la partie aryle mentionnés ci-dessus,

$R^2$ désigne un groupe alkyle linéaire ou ramifié ayant 1 à 6 atomes de carbone,

$R^3$ est un groupe dialkylamino avec 1 à 6 atomes de carbone dans chacune des parties alkyle individuelles identiques ou différentes, linéaires ou ramifiées, ou un reste $-Z-R^4$,

Y représente l'oxygène, le soufre ou un reste

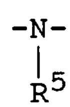

$R^4$ est un groupe alkyle à chaîne droite ou ramifiée ayant 1 à 6 atomes de carbone ou un groupe aralkyle ayant 1 à 4 atomes de carbone dans la partie alkyle linéaire ou ramifiée et 6

à 10 atomes de carbone dans la partie aryle, non substitué ou portant dans la partie aryle un ou plusieurs substituants identiques ou différents, en considérant comme substituants de la partie aryle ceux qui ont été mentionnés pour $R^1$,

$R^5$ est l'hydrogène ou un groupe alkyle linéaire ou ramifié ayant 1 à 6 atomes de carbone et

Z représente l'oxygène ou le soufre, leurs isomères et leurs mélanges d'isomères.

**2.** Esters d'acide acrylique à substituant thiadiazole de formule (I) suivant la revendication 1, dans lesquels

$R^1$ représente l'hydrogène, un groupe méthyle, éthyle, n-propyle, isopropyle, n-butyle, isobutyle, sec.butyle, tertiobutyle, un groupe alkyle de 1 à 4 atomes de carbone linéaire ou ramifié portant 1 à 6 substituants fluoro et chloro identiques ou différents ou un groupe alkyle linéaire ou ramifié ayant 1 à 4 atomes de carbone, qui est substitué par un reste phénylthio ou phényloxy, chacun non substitué ou portant 1 à 3 substituants identiques ou différents, en considérant comme substituants de la partie phényle les substituants indiqués plus loin dans la définition de $R^1$ ;

$R^1$ est un groupe allyle, n-butényle ou isobutényle, un groupe benzyle, phényléthyle, phényléthényle, phényle, naphtyle, pyridile, thiényle ou furyle dont chacun porte le cas échéant dans la partie aryle ou dans la partie hétéroaryle 1 à 3 substituants identiques ou différents, en considérant dans chaque cas comme substituants :
le fluor, le chlore, le brome, un radical cyano, nitro, méthyle, éthyle, n-propyle, isopropyle, n-butyle, isobutyle, sec.butyle, tertiobutyle, méthoxy, éthoxy, n-propoxy, isopropoxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, but-2-ène-di-1,4-yle, 1,4-butanediyle ou un reste phényle, benzyle, phénoxy, ou benzyloxy, chacun étant non substitué ou portant dans la partie phényle 1 à 3 substituants, identiques ou différents, fluoro, chloro, bromo, méthyle, éthyle, méthoxy, éthoxy, méthylthio, trifluorométhyle, difluorométhoxy, trifluorométhoxy et trifluorométhylthio,

$R^2$ est un groupe méthyle, éthyle, n-propyle, isopropyle, n-butyle, isobutyle, sec.butyle ou tertiobutyle,

$R^3$ est un groupe dialkylamino avec 1 à 4 atomes de carbone dans chacune des parties alkyle individuelles linéaires ou ramifiées, identiques ou différentes, ou un reste $-Z-R^4$,

Y représente l'oxygène, le soufre ou un reste

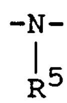

$R^4$ est un groupe méthyle, éthyle, n-propyle, isopropyle, n-butyle, isobutyle, sec.butyle ou tertiobutyle ou un groupe benzyle non substitué ou portant 1 à 3 substituants identiques ou différents, en considérant comme substituants ceux qui ont été mentionnés pour $R^1$ ;

$R^5$ est l'hydrogène ou un groupe méthyle, éthyle, n-propyle, isopropyle, n-butyle, isobutyle, sec.butyle ou tertiobutyle et

Z représente l'oxygène ou le soufre.

**3.** Esters d'acide acrylique à substituant thiadiazole de formule (I) suivant la revendication 1, dans lesquels

$R^1$ représente l'hydrogène, un groupe méthyle, éthyle, n-propyle, isopropyle, tertiobutyle, un groupe alkyle de 1 ou 2 atomes de carbone portant 1 à 5 substituants fluoro et chloro identiques ou différents ou un groupe alkyle ayant 1 ou 2 atomes de carbone, qui est substitué par des radicaux phénylthio et phényloxy, non substitués chacun ou portant chacun 1 ou 2 substituants identiques ou différents, et on mentionne comme substituants de la partie phényle les substituants indiqués plus loin dans la définition de $R^1$, ou bien

$R^1$ est un groupe phényle ou un groupe naphtyle portant chacun le cas échéant 1 à 3 substituants identiques ou différents, en considérant dans chaque cas comme substituants :
le fluor, le chlore, le brome, un radical cyano, nitro, méthyle, éthyle, n-propyle, isopropyle, n-butyle, isobutyle, sec.butyle, tertiobutyle, méthoxy, éthoxy, n-propoxy, isopropoxy, méthylthio,

trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle, cyclohexyle, 1,3-propanediyle, 1,4-butandiyle ou un groupe phényle, phénoxy, benzyle ou benzyloxy, portant éventuellement dans chaque cas 1 ou 2 substituants fluoro, chloro, bromo, méthyle et éthyle identiques ou différents,

$R^2$ est un groupe méthyle ou éthyle,

$R^3$ est un groupe diméthylamino, diéthylamino ou un reste $-Z-R^4$,

Y est l'oxygène, le soufre ou un reste

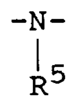

$R^4$ est un groupe méthyle, éthyle, n-propyle, isopropyle, ou benzyle,

$R^5$ est l'hydrogène ou un groupe méthyle ou éthyle, et

Z est l'oxygène ou le soufre.

**4.** Esters d'acide acrylique à substituant thiadiazole de formule (I) suivant la revendication 1, dans lesquels

$R^1$ représente l'hydrogène, un groupe méthyle, éthyle, n-propyle, isopropyle, tertiobutyle, un groupe méthyle portant 1 à 3 substituants fluoro et chloro identiques ou différents ou un groupe phényle, phényloxyméthyle ou phénylthiométhyle portant chacun le cas échéant un ou deux substituants identiques ou différents, en considérant comme substituants :
le fluor, le chlore, le brome, un radical cyano, nitro, méthyle, éthyle, méthoxy, éthoxy, méthylthio, trifluorométhyle, trifluorométhoxy, trifluorométhylthio, méthoxycarbonyle, éthoxycarbonyle, méthoximinométhyle, cyclopentyle, 1,3-propanediyle, méthoximinoéthyle ou un groupe phényle, phénoxy, benzyle ou benzyloxy portant le cas échéant un ou deux substituants fluoro, chloro, bromo ou méthyle identiques ou différents,

$R^2$ est un groupe méthyle ou éthyle,

$R^3$ est un groupe méthoxy, éthoxy, méthylthio ou diméthylamino, et

Y est un reste N-méthyle ou le soufre.

**5.** Procédé de production d'esters d'acide acrylique à substituant thiadiazole de formule générale (I),

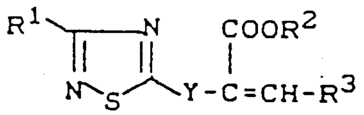

(I)

dans laquelle
$R^1$, $R^2$ et $R^3$ ont la définition indiquée dans la revendication 1,
caractérisé en ce que

(a) on obtient des esters d'acide acrylique à substituant thiadiazole de formule (Ia)

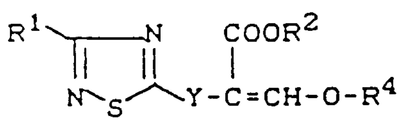

(Ia)

dans laquelle
$R^1$, $R^2$, $R^4$ et Y ont la définition indiquée ci-dessus,
en faisant réagir des esters d'acides hydroxyacryliques ou leurs sels de métaux alcalins de formule (II),

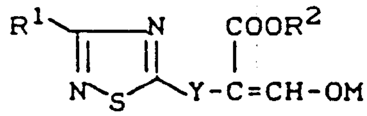

(II)

dans laquelle

M est l'hydrogène ou un cation de métal alcalin et

$R^1$, $R^2$, $R^4$ et Y ont la définition indiquée ci-dessus,

avec des agents alkylants de formule (III),

$R^4$-$E^1$ (III)

dans laquelle

$E^1$ est un groupe partant attirant les électrons et

$R^4$ a la définition indiquée ci-dessus, le cas échéant en présence d'un diluant et en la présence éventuelle d'une substance auxiliaire de réaction, ou bien

(b) on obtient des esters d'acide acrylique à substitutant thiadiazole de formule (Ib)

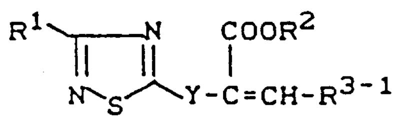

(Ib)

dans laquelle

$R^{3-1}$ est un groupe dialkylamino et

$R^1$, $R^2$ et Y ont la définition indiquée ci-dessus,

en faisant réagir des esters d'acide acétique substitués de formule (IV),

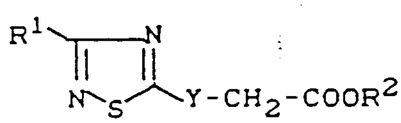

(IV)

dans laquelle

$R^1$, $R^2$ et Y ont la définition indiquée ci-dessus,

avec des dérivés de formamide de formule (V),

$$\begin{matrix}R^7\\R^6\end{matrix} > CH\text{-}R^{3-1} \qquad (V)$$

dans laquelle

$R^6$ et $R^7$ représentent chacun, indépendamment l'un de l'autre, un groupe alkoxy ou dialkylamino et

$R^{3-1}$ a la définition indiquée ci-dessus,

le cas échéant en présence d'un diluant, ou bien

(c) on obtient un ester d'acide acrylique à substituant thiadiazole de formule (Ic),

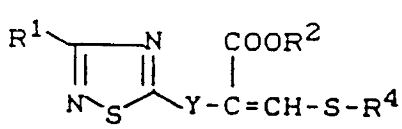

(Ic)

dans laquelle

$R^1$, $R^2$, $R^4$ et Y ont la définition indiquée ci-dessus,
en faisant réagir des esters d'acide acrylique substitués de formule (IV),

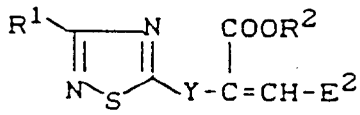

(VI)

dans laquelle

| | |
|---|---|
| $E^2$ | est un groupe partant attirant les électrons et |
| $R^1$, $R^2$ et Y | ont la définition indiquée ci-dessus, avec des thiols de formule (VII) |

$R^4$-SH (VII)

dans laquelle

| | |
|---|---|
| $R^4$ | a la définition indiquée ci-dessus, le cas échéant en présence d'un diluant et en la présence éventuelle d'une substance auxiliaire de réaction. |

**6.** Compositions fongicides, caractérisées par une teneur en au moins un ester d'acide acrylique à substituant thiadiazole de formule (I) suivant les revendications 1 à 5.

**7.** Procédé pour combattre des champignons, caractérisé en ce qu'on fait agir des esters d'acide acrylique à substituants thiadiazole de formule (I) suivant les revendications 1 à 6 sur les champignons et/ou sur leur milieu.

**8.** Utilisation d'esters d'acide acrylique à substituant thiadiazole de formule (I) suivant les revendications 1 à 6 pour combattre des champignons.

**9.** Procédé de préparation de compositions fongicides, caractérisé en ce qu'on mélange des esters d'acide acrylique à substituant thiadiazole de formule (I) suivant les revendications 1 à 6 avec des diluants et/ou des substances tensio-actives.

**10.** Esters d'acides hydroxyacryliques de formule (II)

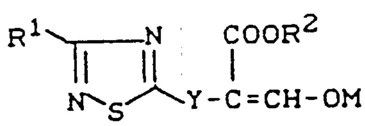

(II)

dans laquelle

| | |
|---|---|
| $R^1$ | et $R^2$ ont la définition indiquée dans la revendication 1 et |
| M | représente l'hydrogène ou un cation de métal alcalin. |

**11.** Procédé de production d'esters d'acides hydroxyacryliques de formule (II) suivant la revendication 10, caractérisé en ce qu'on fait réagir des esters d'acide acétique à substituant thiadiazole de formule (IV)

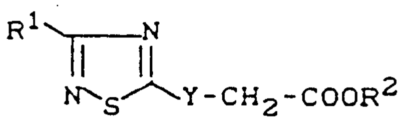

(IV)

dans laquelle
$R^1$, $R^2$ et Y ont la définition indiquée dans la revendication 10,
avec des esters d'acide formique de formule (VIII)

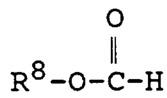

(VIII)

dans laquelle
$R^8$ est un groupe alkyle,
le cas échéant en présence d'un diluant et en la présence éventuelle d'une substance auxiliaire basique de réaction à des températures comprises entre -20 °C et +50 °C puis on effectue éventuellement une hydrolyse avec un acide.

**12.** Esters d'acide acétique à substituant thiadiazole de formule (IVa)

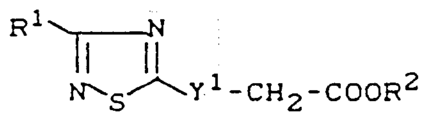

(IVa)

dans laquelle

| | |
|---|---|
| $Y^1$ | représente l'oxygène ou un reste N-alkyle, |
| $R^1$ et $R^2$ | ont la définition indiquée dans la revendication 1, |

excepté le composé ester éthylique de N-méthyl-N-[3-(trichlorométhyl)-1,2,4-thiadiazole-5-yl]glycine.

**13.** Procédé de production d'esters d'acide acétique à substituant thiadiazole de formule (IVa) suivant la revendication 12, caractérisé en ce qu'on fait réagir des dérivés de 1,2,4-thiadiazole de formule (IV),

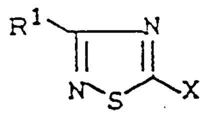

(IX)

dans laquelle

| | |
|---|---|
| $R^1$ | a la définition indiquée dans la revendication 12, et |
| X | est un halogène, |

avec des dérivés d'esters d'acide acétique de formule (X)

$HY^1\text{-}CH_2\text{-}COOR^2$ (X)

dans laquelle
$R^2$ et $Y^1$ ont la définition indiquée dans la revendication 12 ou leurs chlorhydrates,
le cas échéant en présence d'un diluant et en la présence éventuelle d'une base.

**14.** Esters d'acide acrylique substitués de formule (VI)

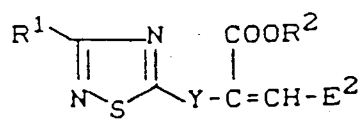

(VI)

dans laquelle

| | |
|---|---|
| $R^1$ et $R^2$ | ont la définition indiquée dans la revendication 1, |
| Y | représente l'oxygène, le soufre ou un reste |

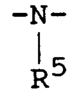

où

$R^5$ représente l'hydrogène ou un groupe alkyle, et

$E^2$ est un groupe partant attirant les électrons.

**15.** Procédé de production d'esters d'acide acrylique substitués de formule (VI) suivant la revendication 14, caractérisé en ce qu'on fait réagir des esters d'acides hydroxyacryliques de formule (II)

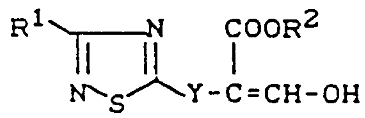
(II)

dans laquelle
$R^1$, $R^2$ et Y ont la définition indiquée dans la revendication 14,
avec des chlorures d'acides de formule (XI),

$R^9$-Cl (XI)

dans laquelle
$R^9$ est un reste acyle ou sulfonyle,
le cas échéant en présence d'un diluant et en la présence éventuelle d'un accepteur d'acide, à des températures comprises entre -20 °C et +120 °C.

**16.** Utilisation de composés de formules (II), (IVa) et (VI) suivant les revendications 10 à 15 comme produits intermédiaires.